(19) Europäisches Patentamt / European Patent Office / Office européen des brevets

(11) **EP 1 362 597 B2**

(12) **NEW EUROPEAN PATENT SPECIFICATION**
After opposition procedure

(45) Date of publication and mention
of the opposition decision:
**25.06.2014 Bulletin 2014/26**

(45) Mention of the grant of the patent:
**24.02.2010 Bulletin 2010/08**

(21) Application number: **03075681.1**

(22) Date of filing: **30.06.1999**

(51) Int Cl.:
*A61K 38/45* (2006.01)   *C12N 9/12* (2006.01)
*A61K 39/39* (2006.01)   *C12N 5/0783* (2010.01)
*A61P 35/00* (2006.01)

(54) **Antigenic peptides derived from telomerase**

Von der Telomerase abgeleitete antigene Peptide

Peptides antigènes dérivés de la télomérase

(84) Designated Contracting States:
**AT BE CH CY DE DK ES FI FR GB GR IE IT LI LU MC NL PT SE**

(30) Priority: **08.07.1998 NO 983141**

(43) Date of publication of application:
**19.11.2003 Bulletin 2003/47**

(60) Divisional application:
**10154215.7 / 2 258 383**
**10183808.4 / 2 316 476**

(62) Document number(s) of the earlier application(s) in accordance with Art. 76 EPC:
**99928238.7 / 1 093 381**

(73) Proprietor: **GemVax AS**
**0273 Oslo (NO)**

(72) Inventors:
• **Moller, Mona**
  **3925 Porsgrunn (NO)**
• **Eriksen, Jon Amund**
  **3916 Porsgrunn (NO)**
• **Gaudernack, Gustav**
  **0310 Oslo (NO)**
• **Gjertsen, Marianne Klemp**
  **0310 Oslo (NO)**
• **Saeterdal, Ingvil**
  **0310 Oslo (NO)**
• **Saeboe-Larsen, Stein**
  **0310 Oslo (NO)**

(74) Representative: **Inspicos A/S**
**Kogle Allé 2**
**P.O. Box 45**
**2970 Hørsholm (DK)**

(56) References cited:
**WO-A-98/14593      WO-A-98/21343**

• **VONDERHEIDE R H ET AL: "THE TELOMERASE CATALYTIC SUBUNIT IS A WIDELY EXPRESSED TUMOR- ASSOCIATED ANTIGEN RECOGNIZED BY CYTOTOXIC T LYMPHOCYTES" IMMUNITY, CELL PRESS, US, vol. 10, no. 6, June 1999 (1999-06), pages 673-679, XP000890114 ISSN: 1074-7613**
• **NAIR S.K. ET AL: 'Induction of cytotoxic T cell responses and tumor immunity against unrelated tumors using telomerase reverse transcriptase RNA transfected dendritic cells' NATURE MEDICINE vol. 6, no. 8, September 2000, pages 1011 - 1017**

EP 1 362 597 B2

**Description**

This invention relates to peptides which elicit T cell mediated immunity, and to cancer vaccines and compositions for anti-cancer treatment comprising such peptide fragments. This invention also relates to pharmaceutical compositions comprising the peptides and methods for generating T lymphocytes capable of recognising and destroying tumour cells in a mammal.

Cancer develops through a multistep process involving several mutational events. These mutations result in altered expression/function of genes belonging to two categories: oncogenes and tumour suppressor genes. Oncogenes arise in nature from proto-oncogenes through point mutations or translocations, thereby resulting in a transformed state of the cell harbouring the mutation. All oncogenes code for and function through a protein. Proto-oncogenes are normal genes of the cell which have the potential of becoming oncogenes. In the majority of cases, proto-oncogenes have been shown to be components of signal transduction pathways. Oncogenes act in a dominant fashion. Tumour-suppressor genes on the other hand, act in a recessive fashion, i.e. through loss of function, and contribute to oncogenesis when both alleles encoding the functional protein have been altered to produce non-functional gene products.

The concerted action of a combination of altered oncogenes and tumour-suppressor genes results in cellular transformation and development of a malignant phenotype.

Such cells are however prone to senescence and have a limited life-span. In the majority of cancers, immortalisation of the tumour cells requires the turning on of an enzyme complex called telomerase. In somatic cells the catalytic subunit of this enzyme is normally not expressed. Additional events, such as the action of proteins encoded by a tumour virus or demethylation of silenced promoter sites can result in expression of a functional telomerase complex in tumour cells.

In the field of human cancer immunology, the last two decades have seen intensive efforts to characterise genuine cancer specific antigens. In particular, effort has been devoted to the analysis of antibodies to human tumour antigens. The prior art suggests that such antibodies can be used for diagnostic and therapeutic purposes, for instance in connection with an anti-cancer agent. However, antibodies can only bind to tumour antigens that are exposed on the surface of tumour cells. For this reason, the efforts to produce a cancer treatment based on the immune system of the body has been less successful than expected.

A fundamental feature of the immune system is that it can distinguish self from nonself and does not normally react against self molecules. It has been shown that rejection of tissues or organs grafted from other individuals is an immune response to the foreign antigens on the surface of the grafted cells. The immune response in general consists of a humeral response, mediated by antibodies, and a cellular response. Antibodies are produced and secreted by B lymphocytes, and typically recognise free antigen in native conformation. They can therefore potentially recognise almost any site exposed on the antigen surface. In contrast to antibodies, T cells, which mediate the cellular arm of the immune response, recognise antigens only in the context of MHC molecules, and only after appropriate antigen processing. This antigen processing usually consists of proteolytic fragmentation of the protein, resulting in peptides that fit into the groove of the MHC molecules. This enables T cells to also recognise peptides derived from intracellular antigens.

T cells can recognise aberrant peptides derived from anywhere in the tumour cell, in the context of MHC molecules on the surface of the tumour cell. The T cells can subsequently be activated to eliminate the tumour cell harbouring the aberrant peptide. In experimental models involving murine tumours it has been shown that point mutations in intracellular "self" proteins may give rise to tumour rejection antigens, consisting of peptides differing in a single amino acid from the normal peptide. The T cells recognising these peptides in the context of the major histocompatibility (MHC) molecules on the surface of the tumour cells are capable of killing the tumour cells and thus rejecting the tumour from the host (Boon et al.,1989, Cell 58, 293-303).

MHC molecules in humans are normally referred to as HLA (human leucocyte associated antigen) molecules. There are two principal classes of HLA molecules, class I and class II. HLA class I molecules are encoded by HLA A, B and C subloci and primarily activate CD8+ cytotoxic T cells. HLA class II molecules, on the other hand, primarily activate CD4+ T cells, and are encoded by the DR, DP and DQ subloci. Every individual normally has six different HLA class I molecules, usually two alleles from each of the three subgroups A, B and C, although in some cases the number of different HLA class I molecules is reduced due to the occurrence of the same HLA allele twice.

The HLA gene products are highly polymorphic. Different individuals express distinct HLA molecules that differ from those found in other individuals. This explains the difficulty of finding HLA matched organ donors in transplantations. The significance of the genetic variation of the HLA molecules in immunobiology is reflected by their role as immune-response genes. Through their peptide binding capacity, the presence or absence of certain HLA molecules governs the capacity of an individual to respond to specific peptide epitopes. As a consequence, HLA molecules determine resistance or susceptibility to disease.

T cells may inhibit the development and growth of cancer by a variety of mechanisms. Cytotoxic T cells, both HLA class I restricted CD8+ and HLA class II restricted CD4+ may directly kill tumour cells presenting the appropriate tumour antigens. Normally, CD4+ helper T cells are needed for cytotoxic CD8+ T cell responses, but if the peptide antigen is presented by an appropriate APC, cytotoxic CD8+ T cells can be activated directly, which results in a quicker, stronger and more efficient response.

While the peptides that are presented by HLA class II molecules are of varying length (12-25 amino acids), the peptides presented by HLA class I molecules must normally be exactly nine amino acid residues long in order to fit into the class I HLA binding groove. A longer peptide will result in non-binding if it cannot be processed internally by an APC or target cell, such as a cancer cell, before presenting in the class I HLA groove. Only a limited number of deviations from this requirement of nine amino acids have been reported, and in those cases the length of the presented peptide has been either eight or ten amino acid residues long.

Reviews of how MHC binds peptides can be found in Hans-Georg Rammensee, Thomas Friede and Stefan Stevanovic, (1995, Immunogenetics, 41, 178-228) and in Barinaga (1992, Science 257, 880-881). Male et al (1987, Advanced Immunology, J.B. Lippincott Company, Philadelphia) offers a more comprehensive explanation of the technical background to this invention.

In our International Application PCT/N092/00032 (published as WO92/14756) we described synthetic peptides and fragments of oncogene protein products which have a point of mutation or translocations as compared to their proto-oncogene or tumour suppressor gene protein. These peptides correspond to, completely cover or are fragments of the processed oncogene protein fragment or tumour suppressor gene fragment as presented by cancer cells or other antigen presenting cells, and are presented as a HLA-peptide complex by at least one allele in every individual. These peptides were also shown to induce specific T cell responses to the actual oncogene protein fragment produced by the cell by processing and presented in the HLA molecule. In particular, we described peptides derived from the p21-ras protein which had point mutations at particular amino acid positions, namely positions 12, 13 and 61. These peptides have been shown to be effective in regulating the growth of cancer cells in vitro. Furthermore, the peptides were shown to elicit CD4+ T cell immunity against cancer cells harbouring the mutated p21-ras oncogene protein through the administration of such peptides in vaccination or cancer therapy schemes. Later we have shown that these peptides also elicit CD8+ T cell immunity against cancer cells harbouring the mutated p21 *ras* oncogene protein through the administration mentioned above (see M.K. Gjertsen et al., Int. J cancer, 1997, vol. 72 p. 784).

However, the peptides described above will be useful only in certain numbers of cancers, namely those which involve oncogenes with point mutations or translocation in a proto-oncogene or tumour suppressor gene. There is therefore a strong need for an anticancer treatment or vaccine which will be effective against a more general range of cancers.

In general, tumours are very heterogeneous with respect to genetic alterations found in the tumour cells. This implies that both the potential therapeutic effect and prophylactic strength of a cancer vaccine will increase with the number of targets that the vaccine is able to elicit T cell immunity against. A multiple target vaccine will also reduce the risk of new tumour formation by treatment escape variants from the primary tumour.

The enzyme telomerase has recently been the focus of attention for its supposed role in prevention of cellular ageing. Telomerase is a RNA-dependent DNA polymerase, which synthesises telomeric DNA repeats using an RNA template that exists as a subunit of the telomerase holoenzyme. The DNA repeats synthesised by the enzyme are incorporated into telomeres, which are specialised DNA-protein structures found at the ends of the linear DNA molecules which make up every chromosome. Telomerase was first identified in the ciliate *Tetrahymena* (Greider and Blackburn, 1985, Cell 43, 405-413). A human telomerase catalytic subunit sequence was recently identified by Meyerson et al (1990, Cell 1197 , 785-795), and Nakamura et al (1997, Science 277, 955-959), who respectively named the gene hEST2 and hTRT. In addition, three other proteins which are associated with telomerase activity have also been identified: p80 and p95 of *Tetrahymena* (Collins et al, 1995, Cell 81, 677-686) and TP1/TLP1, which is the mammalian homologue of *Tetrahymena* p80 (Harrington et al, 1997, Science, 275, 973-977; Nakayama et al., 1997, Cell 88, 875-884).

Telomerase is not expressed in most normal cells in the body. Most somatic lineages in humans show no detectable telomerase activity, but telomerase activity is detected the germline and in some stem cell compartments, which are sites of active cell division (Harley et al., 1994, Cold Spring Harbor Symp. Quant. Biol. 59, 307-315; Kim et al., 1994, Science 266, 2011-2015; Broccoli et al, 1995, PNAS USA 92, 9082-9086; Counter et al., 1995, Blood 85, 2315-2320; Hiyama et al., 1995, J. Immunol. 155, 3711-3715). Telomeres of most types of human somatic cells shorten with increasing age of the organism, consistent with lack of telomerase activity in these cells. Cultured human cells also show telomere shortening. Telomere shortening continues in cultured human cells which have been transformed, until the telomeres have become critically short. At this point, termed the crisis point, significant levels of cell death and karyotypic instability are observed.

Immortal cells, which have acquired the ability to grow indefinitely in culture, emerge at rare frequency from crisis populations. These immortal cells have high levels of telomerase activity and stable telomeres. Telomerase activity is also readily detected in the great majority of human tumour samples analysed to date (Kim et al, 1994, Science 266, 2011-2015), including ovarian carcinoma (Counter et al., 1994, PNAS USA 91, 2900-2904). A comprehensive review is provided by Shay and Bachetti (1997, Eur. J. Cancer 33, 787-791). Thus, activation of telomerase may overcome the barriers to continuous cell division imposed by telomere length. Cells that overcome the normal senescence mechanisms may do so by stabilising telomere length, probably due to the activity of telomerase.

Viruses implicated in human cancer development such as Epstein Barr virus (EBV, related to B cell malignancies and nasopharyngeal carcinomas) and Human Papilloma virus (HPV 16 and 18, related to cervical carcinomas) have long been known to have the capacity to immortalize human cells. It has now been demonstrated that induction of telomerase activity is the key element in this process (Klingelhutz et al, 1996, Nature, 380, 79-82).

Telomerase is therefore a potential target for cancer therapy. Thus, telomerase inhibitors have been proposed as a new class of anti-cancer drugs (reviewed in Sharma et al, 1997, Ann Oncol 8(11), 1063-1074; Axelrod, 1996, Nature Med 2(2), 158-159; Huminiecki, 1996, Acta Biochim Pol, 43(3), 531-538). It has been suggested that the identification of a human telomerase catalytic subunit may provide a biochemical reagent for identifying such drugs (Meyerson et all 1990, Cell 1197, 785-795). Telomerase has also been suggested to be a marker for diagnosis or prognosis of cancer (Soria and Rixe, 1997, Bull Cancer 84(10), 963-970; Dahse et al, 1997, Clin Chem 43(5), 708-714).

As far as we are aware, however, no one has previously suggested that telomerase may function as a useful target for T cell mediated therapy, or that telomerase peptides or proteins may be used for the treatment or prophylaxis of cancer.

WO 98/21343 discloses genes encoding proteins from the telomerase enzyme complex.

WO 98/14593 discloses the amino acid sequence of human telomerase protein and also suggests the use of this protein and certain fragments thereof in active immunotherapy.

In accordance with one aspect of the invention, we provide a telomerase peptide, which consists of between 9 and 25 amino acids wherein the telomerase peptide comprises the sequence of SEQ ID NO: 2, 3, 4, 9, 10 or 12 to 19, or a fragment of SEQ ID NO: 2, 3, 4, 9, 10 or 12 to 19, at least 8 amino acids long, the fragment being capable of inducing a T-cell response.

In accordance with a second aspect of the invention, there is provided a nucleic acid encoding a telomerase peptide as provided in the first aspect of this invention.

We provide, in accordance with a third aspect of this invention a pharmaceutical composition comprising at least one telomerase peptide or nucleic acid as provided in the first or second aspect of this invention and a pharmaceutically acceptable carrier or diluent.

According to a fourth aspect of this invention, we provide a method for the preparation of a pharmaceutical composition as provided in the third aspect of the invention, the method comprising mixing at least one telomerase peptide or nucleic acid as provided in the first or second aspect of the invention with a pharmaceutically acceptable carrier or diluent.

There is further provided, according to a fifth aspect of this invention a pharmaceutical composition comprising a combination of at least one telomerase peptide as provided in the first aspect of this invention and at least one peptide capable of inducing a T cell response against an oncogene or mutant tumour suppressor protein or peptide, together with a pharmaceutically acceptable carrier or diluent.

We further provide, in accordance with a sixth aspect of this invention, a method for the preparation of a pharmaceutical composition as provided in the fifth aspect of this invention, the method comprising mixing at least one telomerase peptide provided in the first aspect of this invention, with at least one peptide capable of inducing a T cell response against an oncogene or tumour suppressor protein or peptide, and a pharmaceutically acceptable carrier or diluent.

In accordance with a seventh aspect of the invention, we provide the use of a peptide for the manufacture of a medicament for the treatment or prophylaxis of cancer, the peptide consisting of the sequences of any one of SEQ ID NO: 12 to 19, the treatment or prophylaxis comprising generating a T cell response, the response being against a peptide consisting of the sequence of one of SEQ ID NO: 12 to 19, respectively, or a fragment thereof, at least 8 amino acids long, producible after processing by an antigen presenting cell.

In accordance with an eighth aspect of the invention, we provide use of a nucleic acid for the manufacture of a medicament for the treatment or prophylaxis of cancer, in which the nucleic acid encodes a peptide consisting of the sequence of any one of SEQ ID NO: 12 to 19, the treatment or prophylaxis comprising generating a T cell response, the response being against a peptide consisting of the sequence of one of SEQ ID NO: 12 to 19, respectively, or a fragment thereof, at least 8 amino acids long, producible after processing by an antigen presenting cell.

In accordance with a ninth aspect of the invention, we provide a method of generating T lymphocytes capable of recognising and destroying tumour cells in a mammal, in which the method comprises culturing a sample of T lymphocytes taken from a mammal in the presence of a peptide in an amount sufficient to generate telomerase specific T lymphocytes, in which the peptides consists of the sequence of any one of SEQ ID NO: 12 to 19, wherein the telomerase specific T lymphocytes generate a response against a peptide consisting of the sequence of one of SEQ ID NOS: 12 to 19, respectively, or a fragment thereof, at least 8 amino acids long, producible after processing by an antigen presenting cell.

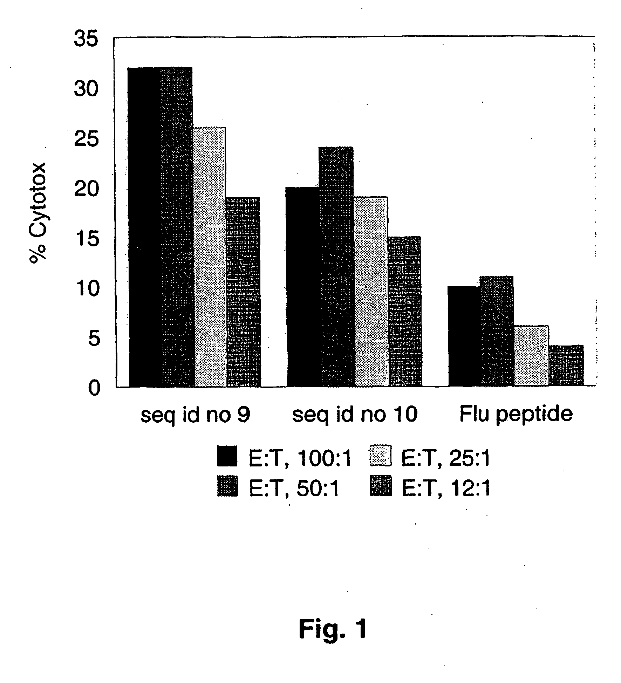

FIGURE 1 shows the sequences of the conserved amino acid motifs in the human telomerase catalytic subunit, as identified by Meyerson et al (1997, Cell 90, 785-795) and Nakamura et al (1997 Science 277, 955-959). Motifs T, 1, 2, 3 (A of Nakamura), 4 (B'of Nakamura) 5 (C of Nakamura), 6 (D of Nakamura) and E are shown. Peptides may be synthesised with sequences corresponding to or encompassing any of the bracketed regions. The designations A2, A1, A3 and B7 indicate peptides which are likely to be presented by HLA-A2, HLA-A1, HLA-A3 and HLA-B7 respectively.

We provide a telomerase peptide for use in a method of treatment or prophylaxis of cancer. The method comprises generating a T cell response against telomerase. The method may comprise administering to a mammal, preferably a human, suffering or likely to suffer from cancer a therapeutically effective amount of the telomerase peptide so that a T cell response against the telomerase is induced in the mammal.

Telomerase specific T cells may be used to target cells which express telomerase. Thus, since most cells in the body of an organism do not express telomerase, they will be unaffected. However, tumour cells that express telomerase will be targeted and destroyed. As telomerase activity has been detected in the majority of cancers identified so far, we expect our materials and methods to have widespread utility.

Cancers which are suitable for treatment include, but are not limited to, breast cancer, prostate cancer, pancreatic cancer, colo-rectal cancer, lung cancer, malignant melanoma, leukaemias, lymphomas, ovarian cancer, cervical cancer and biliary tract carcinomas.

As used here, the term telomerase denotes a ribonucleoprotein enzyme which has telomere elongating activity. Telomerase protein as used here denotes any protein component of telomerase, including any subunit having catalytic activity.

Preferably the telomerase protein is a mammalian telomerase protein, and most preferably a human telomerase protein. The human telomerase protein is preferably the telomerase catalytic subunit identified as hTRT by Nakamura et al (1997, Science 277, 955-959) and hEST2 by Meyerson et al (1990, Cell 1197 , 785-795), the cDNA sequences of which are deposited as GenBank accession numbers AF015950 and AFO18167 respectively.

The term telomerase peptide as used here means a peptide which has an amino acid sequence corresponding to a sequence present in the amino acid sequence of a telomerase protein. The telomerase peptides contain between 9 and 25 amino acids. For instance, the telomerase peptides contain 9, 12, 13, 16 or 21 amino acids.

The telomerase peptide is chosen so that it is capable of generating a T cell response directed against the telomerase protein (or against the telomerase protein from which the telomerase peptide is derived). The T cell response induced is a cytotoxic T cell response. The cytotoxic T cell response may be a CD4+ T cell response, or it may be a CD8+ T cell response. In any case, the peptide must be capable of being presented as a complex with a MHC class I or class II protein on the surface of tumour cells or antigen presenting cells, with antigen processing taking place beforehand if necessary.

The telomerase peptide may include one or more amino acid residues from an amino acid motif essential for the biological function of the telomerase protein; in other words, it may overlap at least partially with such an amino acid motif. Examples of such amino acid motifs are motifs 1 to 6 of the human telomerase catalytic subunit sequence hEST2 as identified by Meyerson et al (1990, Cell 1197, 785-795), in other words, from the motifs

LLRSFFYVTE
SRLRFIPK,
LRPIVNMDYWG,
PELYFVKVDVTGAYDTI,
KSYVQCQGIPQGSILSTLLCSLCY,
LLLRLVDDFLLVT and
GCVVNLRKTVV
or from any of motifs T, 1, 2, A, B', C, D or E as identified by Nakamura et al (1997, Science 277, 955-959) in the hTRT sequence, namely, the motifs

WLMSVYVVELLRSFFYVTETTFQKNRLFFYRKSVWSKLQSIGIRQHLK,
EVRQHREARPALLTSRLRFIPKPDG,
LRPIVNMDYVVGRRTFRREKRAERLTSRV,
PPPELYFVKVDVTGAYDTIPQDRLTEVIASIIKP,
KSYVQCQGIPQGSILSTLLCSLCYGDMENKLFAGI,
LLRLVDDFLLVTPHLTH,
AKTFLRTLVRGVPEYGCWNLRKTVV and HGLFPWCGLLL.

Suitable peptides which may be used in the methods and compositions described here are set out in TABLE 1 as well as in the attached sequence identity list.

Another set of suitable peptides derived from elsewhere in the telomerase sequence, which may be used in the methods and compositions described here, are set out in TABLE 2.

Also included are peptides having amino acid sequences corresponding to an amino acid sequence present in the amino acid sequence of mammalian homologues of the *Tetrahymena* telomerase associated proteins p80 and p95. For example, the p80 homologues TP1 and TLP1 (Harrington et al, 1997, Science, 275, 973-977; Nakayama et al., 1997, Cell 88, 875-884).

The peptides described here are particularly suited for use in a vaccine capable of safely eliciting either CD4+ or CD8+ T cell immunity:

a) the peptides are synthetically produced and therefore do not include transforming cancer genes or other sites or materials which might produce deleterious effects,
(b) the peptides may be used alone to induce cellular immunity,
(c) the peptides may be targeted for a particular type of T cell response without the side effects of other unwanted responses.

The telomerase peptides or proteins described here can be administered in an amount in the range of 1 microgram (1μg) to 1 gram (1g) to an average human patient or individual to be vaccinated. It is preferred to use a smaller dose in the range of 1 microgram (1μg) to 1 milligram (1mg) for each administration.

In preferred embodiments, the telomerase peptide is provided to the patient in the form of a pharmaceutical composition. The telomerase peptide may be administered as a mixture of peptides. The pharmaceutical composition may in addition include the usual additives, diluents, stabilisers or the like as known in the art.

The pharmaceutical composition may comprise one or more telomerase peptides. The peptide mixture may be any one of the following:

(a) a mixture of peptides having different sequences, for example, corresponding to different portions of a telomerase protein sequence;
(b) a mixture of peptides having overlapping sequences, but suitable to fit different HLA alleles;
(c) a mixture of both mixtures (a) and (b);
(d) a mixture of several mixtures (a);
(e) a mixture of several mixtures (b);
(f) a mixture of several mixtures (a) and several mixtures (b);

In each case, a mixture of proteins or peptides corresponding to different telomerase proteins, for example, a telomerase catalytic subunit and a *Tetrahymena* p80 or p95 homologue, may also be used.

The pharmaceutical composition may be made by mixing the or peptide(s) with a pharmaceutically acceptable carrier or diluent.

The pharmaceutical composition may also include at least one peptide capable of inducing a T cell response against an oncogene or mutant tumour suppressor protein or peptide. Alternatively, the telomerase proteins or peptides may be administered either simultaneously or in optional sequence with these peptides. Examples of oncogene proteins are the p21-*ras* proteins H-ras, K-ras and N-ras, abl, gip, gsp, ret and trk. Preferably, the oncogene protein or peptide is a p21-ras protein or peptide, for example, the p21-ras peptides described in our International Application PCT/NO92/00032 (publication number WO92/14756). Tumour suppressor proteins include p53 and Rb (retinoblastoma). Such a pharmaceutical composition may be made by mixing the telomerase protein(s) or peptide(s) with the mutant tumour suppressor or oncogene proteins or peptides, together with a pharmaceutically acceptable carrier or diluent.

As used here, the term mutant refers to a wild type sequence which has one or more of the following: point mutation (transition or transversion), deletion, insertion, duplication translocation or inversion. The term pharmaceutical composition not only encompasses a composition usable in treatment of cancer patients, but also includes compositions useful in connection with prophylaxis, i.e., vaccine compositions.

The telomerase peptides are administered to a human individual in need of such treatment or prophylaxis. The administration may take place one or several times as suitable to establish and/or maintain the wanted T cell immunity. The peptides may be administered together, either simultaneously or separately, with compounds such as cytokines and/or growth factors, i.e., interleukin-2 (IL-2), interleukin-12 (IL-12), granulocyte macrophage colony stimulating factor (GM-CSF) or the like in order to strengthen the immune response as known in the art. The telomerase peptides can be used in a vaccine or a therapeutical composition either alone or in combination with other materials. For example, the peptide or peptides may be supplied in the form of a lipopeptide conjugate which is known to induce a high-affinity cytotoxic T cell response (Deres, 1989, Nature 342).

The peptides mentioned above as possible constituents of the pharmaceutical composition may be provided in the form of nucleic acid encoding the particular peptide. Thus, the pharmaceutical composition may consist of peptide alone, or in combination with nucleic acid, or it may consist of mixtures of nucleic acids.

The telomerase peptides may be administered to an individual in the form of DNA vaccines. The DNA encoding the telomerase peptide may be in the form of cloned plasmid DNA or synthetic oligonucleotide. The DNA may be delivered together with cytokines, such as IL-2, and/or other co-stimulatory molecules. The cytokines and/or co-stimulatory molecules may themselves be delivered in the form of plasmid or oligonucleotide DNA.

The response to a DNA vaccine has been shown to be increased by the presence of immunostimulatory DNA sequences (ISS). These can take the form of hexameric motifs containing methylated CpG, according to the formula : 5'-purine-purine-CG-pyrimidine-pyrimidine-3'. Our DNA vaccines may therefore incorporate these or other ISS, in the DNA encoding the telomerase peptide or protein, in the DNA encoding the cytokine or other co-stimulatory molecules, or in both. A review of the advantages of DNA vaccination is provided by Tighe et al (1998, Immunology Today, 19(2), 89-97).

We describe a method of treatment of a patient afflicted with cancer, the method comprising eliciting T-cell responses through stimulating *in vivo* or *ex vivo* with a telomerase peptide. The telomerase peptide can also be used in a method of vaccination of a patient in order to obtain resistance against cancer. A suitable method of vaccination comprises eliciting T-cell responses through stimulating *in vivo* or *ex vivo* with a telomerase protein or peptide. We also describe a method of treatment or prophylaxis of cancer, comprising administering to a mammal suffering or likely to suffer from cancer a therapeutically effective amount of a telomerase peptide so that a T cell response against telomerase is induced in the mammal.

The peptides described here may be produced by conventional processes, for example, by the various peptide synthesis methods known in the art. Alternatively, they may be fragments of a telomerase protein produced by cleavage, for example, using cyanogen bromide, and subsequent purification. Enzymatic cleavage may also be used. The telomerase peptides may also be in the form of recombinant expressed proteins or peptides.

Nucleic acids encoding the telomerase peptide can be made by oligonucleotide synthesis. This may be done by any of the various methods available in the art. A nucleic acid encoding telomerase protein may be cloned from a genomic or cDNA library, using conventional library screening. The probe may correspond to a portion of any sequence of a known telomerase gene. Alternatively, the nucleic acid can be obtained by using the Polymerase Chain Reaction (PCR). The nucleic acid is preferably DNA, and may suitably be cloned into a vector. Subclones may be generated by using suitable restriction enzymes. The cloned or subcloned DNA may be propagated in a suitable host, for example a bacterial host. Alternatively, the host can be a eukaryotic organism, such as yeast or baculovirus. The telomerase peptides may be produced by expression in a suitable host. In this case, the DNA is cloned into an expression vector. A variety of commercial expression kits are available. The methods described in Maniatis et al (1991, Molecular Cloning: A Laboratory Manual, Cold Spring Harbor, New York, Cold Spring Harbor Laboratory Press) and Harlow and Lane (1988, Antibodies: A Laboratory Manual, Cold Spring Harbor, New York, Cold Spring Harbor Laboratory Press) may be used for these purposes.

## Experimental Methods

The peptides were synthesised by using continuous flow solid phase peptide synthesis. N-a-Fmoc-amino acids with appropriate side chain protection were used. The Fmoc-amino acids were activated for coupling as pentafluorophenyl esters or by using either TBTU or diisopropyl carbodiimide activation prior to coupling. 20% piperidine in DMF was used for selective removal of Fmoc after each coupling. Cleavage from the resin and final removal of side chain protection was performed by 95% TFA containing appropriate scavengers. The peptides were purified and analysed by reversed phase (C18) HPLC. The identify of the peptides was confirmed by using electro-spray mass spectroscopy (Finnigan mat SSQ710).

In order for a cancer vaccine and methods for specific cancer therapy based on T cell immunity to be effective, three conditions must be met:

    (a) the peptide is at least 8 amino acids long and is a fragment of a telomerase protein and
    (b) the peptide is capable of inducing, either in its full length or after processing by antigen presenting cell, T cell responses.

The following experimental methods may be used to determine if these three conditions are met for a particular peptide. First, it should be determined if the particular peptide gives rise to T cell immune responses *in vitro.* It will also need to be established if the synthetic peptides correspond to, or are capable after processing to yield, peptide fragments corresponding to peptide fragments occurring in cancer cells harbouring telomerase or antigen presenting cells that have processed naturally occurring telomerase. The specificity of T cells induced *in vivo* by telomerase peptide vaccination may also be determined.

It is necessary to determine if telomerase expressing tumour cell lines can be killed by T cell clones obtained from peripheral blood from carcinoma patients after telomerase peptide vaccination. T cell clones are obtained after cloning of T-cell blasts present in peripheral blood mononuclear cells (PBMC) from a carcinoma patient after telomerase peptide vaccination. The peptide vaccination protocol includes several *in vivo* injections of peptides intracutaneously with GM-CSF or another commonly used adjuvant. Cloning of T cells is performed by plating responding T cell blasts at 5 blasts per well onto Terasaki plates. Each well contains $2 \times 10^4$ autologous, irradiated (30 Gy) PBMC as feeder cells. The cells are propagated with the candidate telomerase peptide at 25 mM and 5 U/ml recombinant interleukin-2 (rIL-2) (Amersham, Aylesbury, UK) in a total volume of 20 mL. After 9 days T cell clones are transferred onto flat-bottomed 96-well plates (Costar, Cambridge, MA) with 1 mg/ml phytohemagglutinin (PHA, Wellcome, Dartford, UK), 5 U/ml rIL-2 and allogenic irradiated (30 Gy) PBMC (2 x 10^5) per well as feeder cells. Growing clones are further expanded in 24-well plates with PHA / rIL-2 and 1 x 10^6 allogenic, irradiated PBMC as feeder cells and screened for peptide specificity after 4 to 7 days.

T cell clones are selected for further characterisation. The cell-surface phenotype of the T cell clone is determined to ascertain if the T cell clone is CD4+ or CD8+. T cell clone is incubated with autologous tumour cell targets at different effector to target ratios to determine if lysis of tumour cells occurs. Lysis indicates that the T cell has reactivity directed against a tumour derived antigen, for example, telomerase protein.

In order to verify that the antigen recognised is associated with telomerase protein, and to identify the HLA class I or class II molecule presenting the putative telomerase peptide to the T cell clone, different telomerase expressing tumour cell lines carrying one or more HLA class I or II molecules in common with those of the patient are used as target cells in cytotoxicity assays. Target cells are labelled with $^{51}$Cr or $^{3}$H-thymidine (9.25 x 10^4 Bq/mL) overnight, washed once and plated at 5000 cells per well in 96 well plates. T cells are added at different effector to target ratios and the plates are incubated for 4 hours at 37°C and then harvested before counting in a liquid scintillation counter (Packard Topcount). For example, the bladder carcinoma cell line T24 (12Val+, HLA-Al+, B35+), the melanoma cell line FMEX (12Val+, HLA-A2+, B35+) and the colon carcinoma cell line SW 480 (12Val+, HLA-A2', B8+) or any other telomerase positive tumour cell line may be used as target cells. A suitable cell line which does not express telomerase protein may be used as a control, and should not be lysed. Lysis of a particular cell line indicates that the T cell clone being tested recognises an endogenously-processed telomerase epitope in the context of the HLA class I or class II subtype expressed by that cell line.

The HLA class I or class II restriction of a T cell clone may be determined by blocking experiments. Monoclonal antibodies against HLA class I antigens, for example the panreactive HLA class I monoclonal antibody W6/32, or against class II antigens, for example, monoclonals directed against HLA class II DR, DQ and DP antigens (B8/11, SPV-L3 and B7/21), may be used. The T cell clone activity against the autologous tumour cell line is evaluated using monoclonal antibodies directed against HLA class I and class II molecules at a final concentration of 10 mg/ml. Assays are set up as described above in triplicate in 96 well plates and the target cells are preincubated for 30 minutes at 37°C before addition of T cells.

The fine specificity of a T cell clone may be determined using peptide pulsing experiments. To identify the telomerase peptide actually being recognised by a T cell clone, a panel of nonamer peptides is tested. $^{51}$Cr or $^{3}$H-thymidine labelled, mild acid eluted autologous fibroblasts are plated at 2500 cells per well in 96 well plates and pulsed with the peptides at a concentration of 1 mM together with b2-microglobulin (2.5 mg/mL) in a 5% $CO_2$ incubator at 37°C before addition of the T cells. Assays are set up in triplicate in 96 well plates and incubated for 4 hours with an effector to target ratio of 5 to 1. Controls can include T cell clone cultured alone, with APC in the absence of peptides or with an irrelevant melanoma associated peptide MART-1/Melan-A peptide.

An alternative protocol to determine the fine specificity of a T cell clone may also be used. In this alternative protocol, the TAP deficient T2 cell line is used as antigen presenting cells. This cell line expresses only small amounts of HLA-A2 antigen, but increased levels of HLA class I antigens at the cell surface can be induced by addition of b2-microglobulin. $^{3}$H-labelled target cells are incubated with the different test peptides and control peptides at a concentration of 1 mM together with b2-microglobulin (2.5 mg/mL) for one hour at 37°C. After peptide pulsing, the target cells are washed extensively, counted and plated at 2500 cells per well in 96 well plates before addition of the T cells. The plates are incubated for 4 hours at 37°C in 5% $CO_2$ before harvesting. Controls include T cell clone cultured alone or with target cells in the absence of peptides. Assays were set up in triplicate in 96 well plates with an effector to target ratio of 20 to 1.

The sensitivity of a T cell clone to a particular peptide identified above may also be determined using a dose-response experiment. Peptide sensitised fibroblasts can be used as target cells. The target cells are pulsed with the particular peptide as described above for fine specificity determination, with the exception that the peptides are added at different concentrations before the addition of T cells. Controls include target cells alone and target cells pulsed with the irrelevant melanoma associated peptide Melan-A/Mart-1.

Biological experiments/ Description of the figures:

Figure 1

Figure 1 (Fig. 1) describes the induction of telomerase (hTERT) reactive cytotoxic T lymphocytes (CTL's) in HLA-A2(A2/K^b) transgenic mice immunized with telomerase peptides with sequence identity 9 and 10. A standard HLA-A2 restricted influenza (58-66) peptide was used as control. Three groups-of five mice each were given two weekly subcutaneous injections of 10^7 irradiated, peptide pulsed (100 μg/ml) syngeneic spleen cells. One week after the second injection, the mice were sacrificed and their spleens harvested. Spleen cells were prepared by standard techniques, and cells from primed animals were restimulated in vitro for 5 days by coculture with peptide pulsed (10 $\mu$g/ml) irradiated auatologous spleen cells as antigen presenting cells before testing of cytotoxicity against hTERT expressing target cells (Jurkat) transfected with HLA-A2 (A2/K$^b$) in a $^{51}$Cr release assay.

Columns to the left of Fig. 1 show killing of HLA-A2 transfected Jurkat cells pulsed with the control peptide (influenza 58-66) by T cells obtained after priming of mice with the peptide with sequence identity 9, at different effector to target ratios. Specific cytotoxicity above background was observed at all effector to target ratios. Columns in the middle show similar data with T cells obtained from mice primed with the peptide with sequence identity 10. Significant killing of Jurkat cells was only observed when spleen cells from telomerase peptide pulsed mice were used as effector cells, thus when spleen cells from influenza peptide primed mice were used as effectors, only background level of killing of Jurkat cells was seen when the target cells were pulsed with an irrelevant peptide (melanocortin receptor 1 peptide, MC1R244) as evident from columns in the right part of Fig. 1. These results demonstrate that the peptides with sequence identity 9 and 10 are immunogenic in vivo and upon immunization may elicit an immune response in a warm blooded animal carrying the common human MHC molecule HLA-A2. This finding indicates that the peptides with seq. id. no. 9 and 10 may also be used as a cancer vaccine in humans carrying HLA-A2 and other HLA class I molecules capable of binding these peptides. Furthermore, these results demonstrate that hTERT expressed by the T cell leukemia line Jurkat can be processed by the proteolytic machinery of the cell line to yield peptide fragments identical with or similar to the peptides with sequence identity 9 and 10. Together these observations indicate that an immune response obtained after vaccination of cancer patients or patients at risk of developing cancer with these peptides may result in efficient killing of tumor cells expressing the hTERT subunit of telomerase. Fig. 1 depicts cytotoxicity of HLA-A2 transfected Jurkat cells with effector cells obtained from mice immunized as indicated in the figure. Target cells were labeled with $^{51}$Cr (0,1 $\mu$Ci/100 $\mu$l cell suspension) for 1 hr. at 37 °C, washed twice and pulsed with peptide (1 $\mu$g/ml) for 1 hr at 37 °C before washing. Two thousand labeled, peptide pulsed target cells were seeded per well in a 96 well v-bottom microtitre plate, and effector cells (from 2,5x10$^4$ to 2x10$^5$) were added to the wells. Cultures were incubated for 4 hrs. at 37 °C and supernatants were harvested and tested in a gamma-counter. The results in Fig. 1 are expressed as specific cytotoxicity calculated by the following formula:

$$\text{(cpm experimental released - cpm spontaneously released)} /$$
$$\text{(cpm total - cpm spontaneously released)} \times 100$$

Figure 2

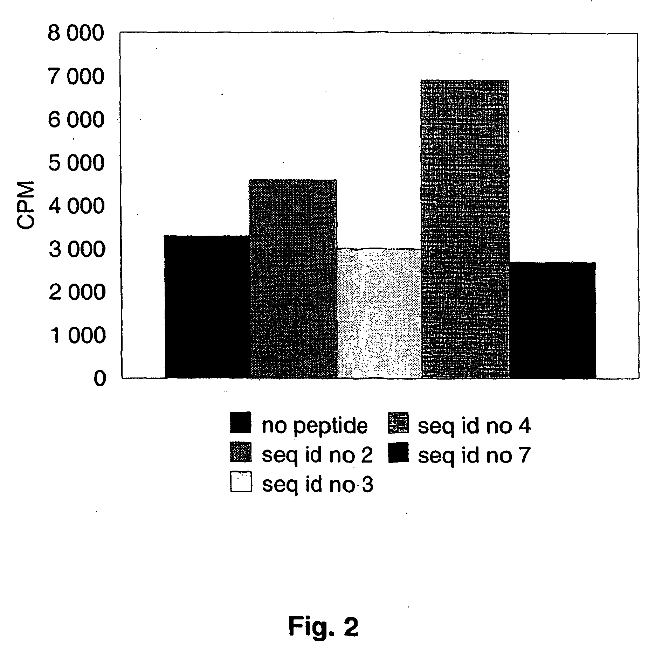

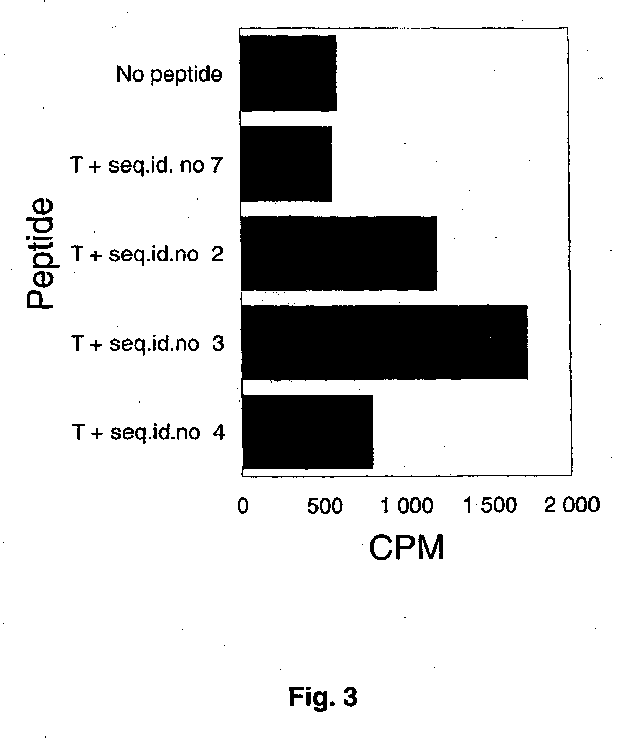

Figure 3 (Fig. 2) shows the results of in vitro stimulation of peripheral blood T cells from a patient (TT) with colon cancer with telomerase (hTERT) derived peptides with sequence identity number 2, 3, 4 and 7. In vitro culture was performed as follows: Triplicates of 10$^5$ mononuclear cells were incubated for 6 days in X-VIVO 10 medium supplemented with 15% pooled heat inactivated human serum in a humidified incubator in 5% CO$_2$. Peptides were present throughout culture at a final concentration of 30 $\mu$g/ml in the medium. Cultures without peptide served as control. A proliferative response above background values was seen when the T cells were stimulated with the peptide with sequence identity 4. These results demonstrate that blood from a cancer patient contains circulating T cells specific for a peptide derived from telomerase (hTERT). These results demonstrate that the enzymatic subunit of telomerase (hTERT) is immunogenic in man, and may spontaneously give rise to telomerase specific T cell responses when overexpressed by a tumor growing in the patient. Furthermore, one component of the telomerase specific response in this patient is directed against the peptide with seq. id. no. 4 described here. This finding indicates that the peptide with seq. id. no. 4 may also be used as a cancer vaccine in humans. The figure depicts the results of conventional T cell proliferative assays, where peripheral blood mononuclear cells (10$^5$) were cultured with peptides as indicated for 7 days in triplicates before harvesting. To measure the proliferative capacity of the cultures, $^3$H-thymidine (3,7x10$^4$ Bq/ well) was added to the culture overnight before harvesting. Values are given as mean counts per minute (cpm) of the triplicates.

Figures 3 and 4

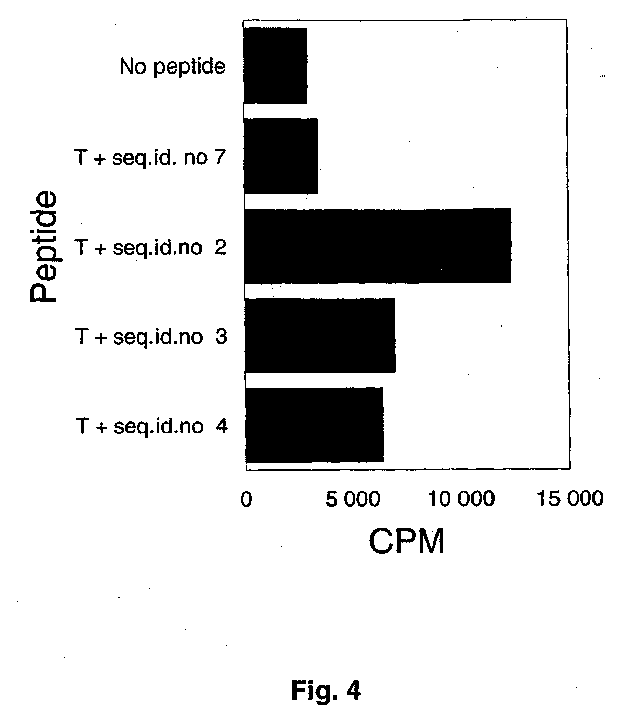

Figures 3 and 4 (Fig. 3 and Fig. 4) show the reactivity of tumor infiltrating lymphocytes (TILs) obtained from a patient with advanced pancreatic cancer. The T cells were obtained from a tumor biopsy and was successfully propagated *in vitro* to establish a T cell line. The T cell line was CD3+, CD4+ and CD8-, and proliferated specificially in response to the telomerase peptides. The results in Fig. 3 show T cells that recognise the peptides with seq. id. no. 2 and 3 when compared to controls with medium alone. The results in Fig. 4 show T cells that recognise the peptide with seq. id. no. 2. The TILs were expanded by co-cultureing with recombinant human interleukin 2 (rIL-2) and tested after 14 days in standard proliferation assay using peptides with sequence id. nos. 2, 3, 4 and 7.

## Table 1

LMSVYWEL     FLHWLMSVYVVELLRSFFYVTE
ELLRSFFYV     EARPALLTSRLRFIPK
YVVELLRSF     DGLRPIVNMDYVVGAR
VVELLRS FF     GVPEYGCVVNLRKVVNF
SVYVVELLR
VELLRSFFY
YVTETTFQK
RLFFYRKSV
SIGIRQHLK
RPALLTSRL
ALLTSRLRF
LLTSRLRFI
RPIVNMDYV
LRPIVNMDY
YVVGARTFR
VVGARTFRR
GARTFRREK
ARTFRREKP
PPELYFVKV
ELYFVKVDV
FVKVDVTGA
IPQDRLTEV
DRLTEVIAS
RLTEVIASI
IPQGSILSTL
ILSTLLCSL
LLRLVDDFL
RLVDDFLLV
VPEYGCVVN
VPEYGCVVNL
TLVRGVPEY
FLRTLVRGV
GVPEYGCVV
VVNLRKTVV
GLFPWCGLL

## Table 2

YAETKHFLY
ISDTASLCY
DTDPRRLVQ
AQDPPPELY
LTDLQPYMR
QSDYSSYAR

ILAKFLHWL
ELLRSFFYV
LLARCALFV
WLCHQAFLL
RLVDDFLLV

(continued)

RLFFYRKSV
LQLPFHQQV
RLGPQGWRL
SLQELTWKM
NVLAFGFAL
VLLKTHCPL
FLLVTPHLT
TLTDLQPYM
RLTEVIASI
FLDLQVNSL
SLNEASSGL
ILSTLLCSL
LLGASVLGL
VLAFGFALL
LQPYMRQFV
LMSVYVVEL
RLPQRYWQM
RQHSSPWQV
YLPNTVTDA
NMRRKLFGV
RLTSRVKAL
LLQAYRFHA
LLDTRTLEV
YMRQFVAHL
LLTSRLRFI
CLVCVPWDA
LLSSLRPSL
FMCHHAVRI
LQVNSLQTV
LVAQCLVCV
CLKELVARV
FLRNTKKFI
ALPSDFKTI
VLVHLLARC
VQSDYSSYA
SVWSKLQSI
KLPGTTLTA
QLSRKLPGT
ELYFVKVDV
GLLLDTRTL
WMPGTPRRL
SLTGARRLV
WIEQSSSL
LPSEAVQWL
QAYRFHACV

GLFDVFLRF
KLFGVLRLK
RLREEILAK
TLVRGVPEY
GLPAPGARR

(continued)

GLFPWCGLL
KLTRHRVTY
VLPLATFVR
ELVARVLQR

DPRRLVQLL
FVRACLRRL
SVREAGVPL
AGRNMRRKL
LARCALFVL
RPAEEATSL
LPSDFKTIL
LPSEAVQWL
LPGTTLTAL
RPSFLLSSL
LPNTVTDAL
RPALLTSRL
RCRAVRSLL
MPRAPRCRA

GIRRDGLLL
VLRLKCHSL
YMRQFVAHL
SLRTAQTQL
QMRPLFLEL
LLRLVDDFL
FVQMPAHGL
HASGPRRRL
VVIEQSSSL
RVISDTASL
CVPAAEHRL
RVKALFSVL
NVLAFGFAL
LVARVLQRL
FAGIRRDGL
HAQCPYGVL
RAQDPPPEL
AYRFHACVL
HAKLSLQEL
GAKGAAGPL
TASLCYSIL
APRCRAVRS
GARRLVETI
AQCPYGVLL
HAKTFLRTL
EATSLEGAL
KAKNAGMSL
AQTQLSRKL
AGIRRDGLL

VLRLKCHSL

(continued)

ILKAKNAGM
DPRRLVQLL
GAKGAAGPL
FAGIRRDGL
GARRRGGSA
HAKTFLRTL
HAKLSLQEL
LARCALFVL
EHRLREEIL
NMRRKLFGV

CAREKPQGS
LTRHRVTYV

RRFLRNTKK
RRDGLLLRL
RREKRAERL
RRLVETIFL
LRFMCHHAV
RRYAWQKA
KRAERLTSR
RRKLFGVLR
RRRGGSASR
RRLPRLPQR
RRLGPQGWR
LRGSGAWGL
HREARPALL
VRRYAVVQK
ARTSIRASL
HRVTYVPLL
LRSHYREVL
MRPLFLELL
HRAWRTFVL
MRRKLFGVL
LRLVDDFLL
LRRVGDDVL
YRKSVWSKL
QRLCERGAK
FRALVAQCL
SRKLPGTTL
LRRLVPPGL
RRSPGVGCV
RRVGDDVLV
VRGCAWLRR
VRSLLRSHY
ARTFRREKR
SRSLPLPKR
IRASLTFNR
KEQLRPSFL
REKPQGSVA
LEVQSDYSS

(continued)

REARPALLT
EEDTDPRRL
REEILAKFL
CERGAKNVL
DDVLVHLLA
GDMENKLFA
YERARRPGL
LREEILAKF
IRRDGLLLR
QRGDPAAFR
LRPIVNMDY

ARRLVETIF
ARPALLTSR
LRPSLTGAR
LRLKCHSLF
FRREKRAER
ARGGPPEAF
CRAVRSLLR
GRTRGPSDR
RRRLGCERA
LRELSEAEV
ARCALFVLV

RPAEEATSL
DPRRLVQLL
RPSFLLSSL
LPSEAVQWL
RPALLTSRL
LPSDFKTIL
RPPPAAPSF
LPRLPQRYW
LPNTVTDAL
LPGTTLTAL
LAKFLHWLM
KAKNAGMSL
GSRHNERRF
KALFSVLNY
SPLRDAWI
RAQDPPPEL
MPAHGLFPW

AEVRQHREA
REAGVPLGL
EEATSLEGA
LEAAANPAL
QETSPLRDA
REVLPLATF

**Claims**

1. A telomerase peptide, which consists of between 9 and 25 amino acids wherein the telomerase peptide comprises the sequence of SEQ ID NO: 2, 3, 4, 9, 10 or 12 to 19, or a fragment of SEQ ID NO: 2, 3, 4, 9, 10 or 12 to 19, at least 8 amino acids long, the fragment being capable of inducing a T cell response.

2. A telomerase peptide according to Claim 1 consisting of the sequence of SEQ ID NO: 2, 3, 4, 9, 10 or 12 to 19.

3. A telomerase peptide according to Claim 1 or 2 for use in a method of treatment or prophylaxis of cancer.

4. A telomerase peptide as claimed in Claim 3 for a use as specified therein, the telomerase peptide being capable of generating a T cell response directed against the telomerase protein.

5. A telomerase peptide as claimed in Claim 3 or 4 for a use as specified therein, in which the method comprises administering to a mammal suffering or likely to suffer from cancer a therapeutically effective amount of the telomerase peptide so that a T cell response against the telomerase is induced in the mammal.

6. A telomerase peptide as claimed in Claim 4 or 5 for a use as specified therein, in which the T cell response induced is a cytotoxic T cell response.

7. A nucleic acid encoding a telomerase peptide according to Claim 1 or 2.

8. A nucleic acid according to Claim 7 for use in a method of treatment or prophylaxis of cancer.

9. A pharmaceutical composition comprising at least one telomerase peptide as claimed in Claim 1 or 2, or at least one nucleic acid as claimed in Claim 7, together with a pharmaceutically acceptable carrier or diluent.

10. A pharmaceutical composition comprising a combination of at least one telomerase peptide as claimed in Claims 1 or 2 and at least one peptide capable of inducing a T cell response against an oncogene or mutant tumour suppressor protein or peptide, together with a pharmaceutically acceptable carrier or diluent.

11. A pharmaceutical composition as claimed in Claim 9 or 10 for use in the treatment or prophylaxis of any of the following cancers: breast cancer, prostate cancer, pancreatic cancer, colo-rectal cancer, lung cancer, malignant melanoma, leukaemias, lymphomas, ovarian cancer, cervical cancer and biliary tract carcinomas.

12. A method for the preparation of a pharmaceutical composition as claimed in Claim 9, in which the method comprises mixing at least one telomerase peptide as claimed in Claim 1 or 2, or at least one nucleic acid as claimed in Claim 7, with a pharmaceutically acceptable carrier or diluent.

13. A method for the preparation of a pharmaceutical composition as claimed in Claim 10, in which the method comprises mixing at least one telomerase peptide as claimed in Claim 1 or 2, with at least one peptide capable of inducing a T cell response against an oncogene or mutant tumour suppressor protein or peptide, and a pharmaceutically acceptable carrier or diluent.

14. A pharmaceutical composition as claimed in Claim 10 or a method of making a pharmaceutical composition as claimed in Claim 13, in which the oncogene protein or peptide is a mutant p21-*ras* protein or peptide.

15. A pharmaceutical composition as claimed in claimed in Claim 10 or a method of making a pharmaceutical composition as claimed in Claim 13, in which the tumour suppressor protein or peptide is a retinoblastoma or p53 protein or peptide.

16. The use of a peptide for the manufacture of a medicament for the treatment or prophylaxis of cancer, the peptide consisting of the sequence of any one of SEQ ID NO: 12 to 19, the treatment or prophylaxis comprising generating a T cell response, the response being against a peptide consisting of the sequence of one of SEQ ID NO: 12 to 19, respectively, or a fragment thereof, at least 8 amino acids long, producible after processing by an antigen presenting cell.

17. The use of a nucleic acid for the manufacture of a medicament for the treatment or prophylaxis of cancer, in which the nucleic acid encodes a peptide consisting of the sequence of any one of SEQ ID NO: 12 to 19, the treatment or prophylaxis comprising generating a T cell response, the response being against a peptide consisting of the sequence of one of SEQ ID NO: 12 to 19, respectively, or a fragment thereof, at least 8 amino acids long, producible after processing by an antigen presenting cell.

18. Use according to Claim 16 or 17, in which the treatment or prophylaxis comprises administering to a mammal suffering or likely to suffer from cancer a therapeutically or prophylactically effective amount of the peptide so that a T cell response directed against the telomerase is induced in the mammal.

19. Use according to any one of Claims 16 to 18 in which the T cell response induced is a cytotoxic T cell response.

20. Use according to any one of Claims 16 to 19 wherein the medicament is a pharmaceutical composition comprising the peptide or nucleic acid, together with a pharmaceutically acceptable carrier or diluent.

21. Use according to any one of Claims 16 or 18 to 20 wherein the medicament comprises a peptide consisting of the sequence of any one of SEQ ID NOS: 12 to 19 and at least one peptide capable of inducing a T cell response directed against an oncogene or mutant tumour suppressor protein or peptide, together with a pharmaceutically acceptable carrier or diluent.

22. Use according to Claim 21 in which the oncogene protein or peptide is a mutant p21-*ras* protein or peptide, or in which the tumour suppressor protein or peptide is a retinoblastoma or p53 protein or peptide.

23. Use according to any one of claims 16 to 22, in which the cancer is selected from breast cancer, prostate cancer, pancreatic cancer, colorectal cancer, lung cancer, malignant melanoma, leukaemias, lymphomas, ovarian cancer, cervical cancer and biliary tract carcinomas.

24. A method of generating T lymphocytes capable of recognising and destroying tumour cells in a mammal, in which the method comprises culturing a sample of T lymphocytes taken from a mammal in the presence of a peptide in an amount sufficient to generate telomerase specific T lymphocytes, in which the peptide consists of the sequence of any one of SEQ ID NO: 12 to 19, wherein the telomerase specific T lymphocytes generate a response against a peptide consisting of the sequence of one of SEQ ID NOS: 12 to 19, respectively, or a fragment thereof, at least 8 amino acids long, producible after processing by an antigen presenting cell.

25. A telomerase specific T lymphocyte generated by a method according to Claim 24.


**Patentansprüche**

1. Telomerasepeptid, welches aus zwischen 9 und 25 Aminosäuren besteht, wobei das Telomerasepeptid die Sequenz von SEQ ID Nr.: 2, 3, 4, 9, 10 oder 12 bis 19 umfasst, oder ein Fragment von SEQ ID Nr.: 2, 3, 4, 9, 10 oder 12 bis 19, mit mindestens einer Länge von 8 Aminosäuren, wobei das Fragment fähig ist eine T-Zell-Antwort zu induzieren.

2. Telomerasepeptid nach Anspruch 1, welches aus der Sequenz von SEQ ID Nr.: 2, 3, 4, 9, 10 oder 12 bis 19 besteht.

3. Telomerasepeptid nach Anspruch 1 oder 2 zur Verwendung in einem Verfahren zur Behandlung oder Prophylaxe von Krebs.

4. Telomerasepeptid gemäß Anspruch 3 für eine Verwendung wie darin angegeben, wobei das Telomerasepeptid fähig ist eine T-Zell-Antwort zu generieren, die gegen das Telomeraseprotein gerichtet ist.

5. Telomerasepeptid gemäß Anspruch 3 oder 4 für eine Verwendung wie darin angegeben, wobei das Verfahren das Verabreichen einer therapeutisch wirksamen Menge des Telomerasepeptids an ein Säugetier umfasst, welches an Krebs erkrankt ist oder voraussichtlich erkranken wird, so dass eine T-Zell-Antwort im Säugetier induziert wird, die gegen die Telomerase gerichtet ist.

6. Telomerasepeptid gemäß Anspruch 4 oder 5 für eine Verwendung wie darin angegeben, wobei die induzierte T-Zell-Antwort eine cytotoxische T-Zell-Antwort ist.

7. Nukleinsäure, die ein Telomerasepeptid nach Anspruch 1 oder 2 codiert.

8. Nukleinsäure nach Anspruch 7 zur Verwendung in einem Verfahren zur Behandlung oder Prophylaxe von Krebs.

9. Pharmazeutische Zusammensetzung, die mindestens ein Telomerasepeptid gemäß Anspruch 1 oder 2 umfasst, oder mindestens eine Nukleinsäure gemäß Anspruch 7, zusammen mit einem pharmazeutisch verträglichen Träger oder Verdünnungsmittel.

10. Pharmazeutische Zusammensetzung, die eine Kombination von mindestens einem Telomerasepeptid gemäß Anspruch 1 oder 2 und mindestens einem Peptid umfasst, welches fähig ist eine T-Zell-Antwort gegen ein Onkogen- oder ein mutiertes Tumorsuppressor-Protein oder -Peptid zu induzieren, zusammen mit einem pharmazeutisch verträglichen Träger oder Verdünnungsmittel.

11. Pharmazeutische Zusammensetzung gemäß Anspruch 9 oder 10 zur Verwendung bei der Behandlung oder Prophylaxe von einem der folgenden Krebsarten: Brustkrebs, Prostatakrebs, Pankreaskrebs, kolorektaler Krebs, Lungenkrebs, malignes Melanom, Leukämien, Lymphome, Eierstockkrebs, Gebärmutterhalskrebs und Gallenwegkarzinome.

12. Verfahren zur Herstellung einer pharmazeutischen Zusammensetzung gemäß Anspruch 9, wobei das Verfahren das Mischen mindestens eines Telomerasepeptids gemäß Anspruch 1 oder 2, oder mindestens einer Nukleinsäure gemäß Anspruch 7, mit einem pharmazeutisch verträglichen Träger oder Verdünnungsmittel umfasst.

13. Verfahren zur Herstellung einer pharmazeutischen Zusammensetzung gemäß Anspruch 10, wobei das Verfahren das Mischen mindestens eines Telomerasepeptids gemäß Anspruch 1 oder 2, mit mindestens einem Peptid umfasst, welches fähig ist eine T-Zell-Antwort gegen ein Onkogen- oder ein mutiertes Tumorsuppressor-Protein oder -Peptid zu induzieren, und einem pharmazeutisch verträglichen Träger oder Verdünnungsmittel.

14. Pharmazeutische Zusammensetzung gemäß Anspruch 10 oder ein Verfahren zur Herstellung einer pharmazeutischen Zusammensetzung gemäß Anspruch 13, wobei das Onkogen-Protein oder-Peptid ein mutiertes p21-ras Protein oder Peptid ist.

15. Pharmazeutische Zusammensetzung gemäß Anspruch 10 oder ein Verfahren zur Herstellung einer pharmazeutischen Zusammensetzung gemäß Anspruch 13, wobei das Tumorsuppressor-Protein oder -Peptid ein Retinoblastom- oder ein p53-Protein oder -Peptid ist.

16. Verwendung eines Peptids zur Herstellung eines Medikaments für die Behandlung oder Prophylaxe von Krebs, wobei das Peptid aus der Sequenz von einer der SEQ ID Nr.: 12 bis 19 besteht, wobei die Behandlung oder Prophylaxe die Generierung einer T-Zell-Antwort umfasst, beziehungsweise die Antwort gegen ein Peptid, das aus der Sequenz von einer der SEQ ID Nr.: 12 bis 19 besteht, oder ein Fragment davon, mit mindestens einer Länge von 8 Aminosäuren, herstellbar nach der Prozessierung durch eine antigenpräsentierende Zelle.

17. Verwendung einer Nukleinsäure zur Herstellung eines Medikaments für die Behandlung oder Prophylaxe von Krebs, wobei die Nukleinsäure ein Peptid codiert, das aus der Sequenz von einer der SEQ ID Nr.: 12 bis 19 besteht, wobei die Behandlung oder Prophylaxe die Generierung einer T-Zell-Antwort umfasst, beziehungsweise die Antwort gegen ein Peptid, das aus der Sequenz von einer der SEQ ID Nr.: 12 bis 19 besteht, oder ein Fragment davon, mit mindestens einer Länge von 8 Aminosäuren, herstellbar nach der Prozessierung durch eine antigenpräsentierende Zelle.

18. Verwendung nach Anspruch 16 oder 17, wobei die Behandlung oder Prophylaxe das Verabreichen einer therapeutisch oder prophylaktisch wirksamen Menge des Telomerasepeptids an ein Säugetier umfasst, welches an Krebs erkrankt ist oder voraussichtlich erkranken wird, so dass eine T-Zell-Antwort im Säugetier induziert wird, die gegen die Telomerase gerichtet ist.

19. Verwendung nach einem der Ansprüche 16 bis 18, wobei die induzierte T-Zell-Antwort eine cytotoxische T-Zell-Antwort ist.

20. Verwendung nach einem der Ansprüche 16 bis 19, wobei das Medikament eine pharmazeutische Zusammensetzung aufweist, welche das Peptid oder Nukleinsäure zusammen mit einem pharmazeutisch verträglichen Träger oder Verdünnungsmittel umfasst.

21. Verwendung nach einem der Ansprüche 16 oder 18 bis 20, wobei das Medikament ein Peptid umfasst, welches aus der Sequenz von einer der SEQ ID Nr.: 12 bis 19 besteht und mindestens einem Peptid, das fähig ist eine T-Zell-Antwort zu induzieren, welche gegen ein Onkogen- oder ein mutiertes Tumorsuppressor-Protein oder-Peptid gerichtet ist, zusammen mit einem pharmazeutisch verträglichen Träger oder Verdünnungsmittel.

22. Verwendung nach Anspruch 21, wobei das Onkogen-Protein oder-Peptid ein mutiertes p21-ras Protein oder Peptid ist, oder wobei das Tumorsuppressor-Protein oder -Peptid ein Retinoblastom- oder ein p53-Protein oder -Peptid ist.

23. Verwendung nach einem der Ansprüche 16 bis 22, wobei der Krebs ausgewählt wird aus Brustkrebs, Prostatakrebs, Pankreaskrebs, kolorektaler Krebs, Lungenkrebs, malignes Melanom, Leukämien, Lymphome, Eierstockkrebs, Gebärmutterhalskrebs und Gallenwegkarzinome.

24. Verfahren zur Generierung von T-Lymphozyten, die fähig sind Tumorzellen in einem Säugetier zu erkennen und zu zerstören, wobei das Verfahren das Kultivieren einer Probe von einem Säugetier entnommenen T-Lymphozyten umfasst, in Gegenwart eines Peptids, das in einer Menge vorliegt, die ausreicht, um telomerasespezifische T-Lymphozyten zu generieren, wobei das Peptid aus der Sequenz von einer der SEQ ID Nr: 12 bis 19 besteht, beziehungsweise wobei die telomerasespezifischen T-Lymphozyten eine Antwort gegen ein Peptid generieren, welches aus der Sequenz von einem der SEQ ID Nr.: 12 bis 19 besteht, oder ein Fragment davon, mit einer Länge von mindestens 8 Aminosäuren, herstellbar nach der Prozessierung durch eine antigenpräsentierende Zelle.

25. Telomerasespezifischer T-Lymphozyt, der durch ein Verfahren nach Anspruch 24 generiert wird.

## Revendications

1. Un peptide de la télomérase, qui est constitué de 9 à 25 acides aminés, dans lequel le peptide de la télomérase comprend la séquence SEQ ID n° 2, 3, 4, 9, 10 ou 12 à 19, ou un fragment de la séquence SEQ ID n° 2, 3, 4, 9, 10 ou 12 à 19, long d'au moins 8 acides aminés, le fragment étant capable d'induire une réponse de la cellule T.

2. Un peptide de la télomérase selon la revendication 1, constitué de la séquence SEQ ID n° 2, 3,4,9, 10 ou 12 à 19.

3. Un peptide de la télomérase selon la revendication 1 ou 2 pour une utilisation dans une méthode de traitement ou de prophylaxie du cancer.

4. Un peptide de la télomérase selon la revendication 3, pour une utilisation comme indiqué dans cette revendication, le peptide de la télomérase étant capable de générer un réponse de la cellule T dirigée contre la protéine de la télomérase.

5. Un peptide de la télomérase selon la revendication 3 ou 4 pour une utilisation comme indiqué dans ces revendications, dans lequel la méthode comprend l'administration à un mammifère souffrant ou susceptible de souffrir du cancer, d'une quantité thérapeutiquement efficace du peptide de la télomérase de sorte qu'une réponse de la cellule T contre la télomérase soit induite dans le mammifère.

6. Un peptide de la télomérase selon la revendication 4 ou 5 pour une utilisation comme indiqué dans ces revendications, dans lequel la réponse induite de la cellule T est une réponse de la cellule T cytotoxique.

7. Un acide nucléique encodant un peptide de la télomérase selon la revendication 1 ou 2.

8. Un acide nucléique selon la revendication 7 pour une utilisation dans une méthode de traitement ou de prophylaxie du cancer.

9. Une composition pharmaceutique comprenant au moins un peptide de la télomérase selon la revendication 1 ou 2, ou au moins un acide nucléique selon la revendication 7, conjointement avec un support ou diluant pharmaceutiquement acceptable.

10. Une composition pharmaceutique comprenant une combinaison d'au moins un peptide de la télomérase selon les revendications 1 ou 2 et au moins un peptide capable d'induire une réponse de la cellule T contre un oncogène ou un peptide ou une protéine supprimant une tumeur mutante, conjointement avec un support ou diluant pharmaceutiquement acceptable.

11. Une composition pharmaceutique selon la revendication 9 ou 10 pour une utilisation dans le traitement ou la prophylaxie d'un des cancers suivants : cancer du sein, cancer de la prostate, cancer du pancréas, cancer coloréctal, cancer du poumon, mélanome malin, leucémie, lymphome, cancer de l'ovaire, cancer de l'utérus et carcinome du tractus biliaire.

12. Un procédé de préparation d'un composition pharmaceutique selon la revendication 9, dans lequel le procédé comprend le mélange d'au moins un peptide de la télomérase selon la revendication 1 ou 2, ou au moins un acide nucléique selon la revendication 7, avec un support ou diluant pharmaceutiquement acceptable.

13. Un procédé de préparation d'une composition pharmaceutique selon la revendication 10, dans laquelle le procédé comprend le mélange d'au moins un peptide de la télomérase selon la revendication 1 ou 2, avec au moins un peptide capable d'induire une réponse de la cellule T contre un oncogène ou un peptide ou une protéine supprimant une tumeur mutante, et un support ou diluant pharmaceutiquement acceptable.

14. Une composition pharmaceutique selon la revendication 10 ou un procédé de fabrication d'une composition pharmaceutique selon la revendication 13, dans lequel le peptide ou la protéine oncogène est un peptide ou une protéine p21-*ras* mutante.

15. Une composition pharmaceutique selon la revendication 10 ou un procédé de fabrication d'une composition pharmaceutique selon la revendication 13, dans lequel le peptide ou la protéine supprimant la tumeur est un peptide ou une protéine p53 ou un rétinoblastome.

16. L'utilisation d'un peptide pour la fabrication d'un médicament pour le traitement ou la prophylaxie du cancer, le peptide étant constitué de l'une quelconque des séquences SEQ ID n° 12 à 19, le traitement ou la prophylaxie comprenant la génération d'une réponse de la cellule T, la réponse étant dirigée contre un peptide constitué de l'une quelconque des séquences SEQ ID n° 12 ou 19, respectivement, ou un fragment de cette séquence, long d'au moins 8 acides aminés, que l'on peut produire après traitement avec une cellule présentant un antigène.

17. L'utilisation d'un acide nucléique pour la préparation d'un médicament pour le traitement ou la prophylaxie du cancer, dans laquelle l'acide nucléique encode un peptide constitué de l'une quelconque des séquences SEQ ID n° 12 à 19, le traitement ou la prophylaxie comprenant la génération d'une réponse de la cellule T, la réponse étant dirigée contre un peptide constitué de l'une quelconque des séquences SEQ ID n° 12 à 19, respectivement, ou un fragment de cette séquence, long d'au moins 8 acides aminés, que l'on peut produire après traitement avec une cellule présentant un antigène.

18. Utilisation selon la revendication 16 ou 17, dans laquelle le traitement ou la prophylaxie comprend l'administration à un mammifère souffrant ou susceptible de souffrir du cancer, d'une quantité efficace thérapeutiquement ou prophylactiquement du peptide de sorte qu'une réponse de la cellule T dirigée contre la télomérase soit induite dans le mammifère.

19. Utilisation selon l'une quelconque des revendications 16 à 18 dans laquelle la réponse de la cellule T induite est une réponse de la cellule T cytotoxique.

20. Utilisation selon l'une quelconque des revendications 16 à 19, dans laquelle le médicament est une composition pharmaceutique comprenant le peptide ou l'acide nucléique, conjointement avec un support ou diluant pharmaceutiquement acceptable.

21. Utilisation selon l'une quelconque des revendications 16 ou 18 à 20 dans laquelle le médicament comprend un peptide constitué de l'une quelconque des séquences SEQ ID n° 12 à 19 et au moins un peptide capable d'induire une réponse de la cellule T dirigée contre un oncogène ou un peptide ou une protéine supprimant une tumeur mutante, conjointement avec un support ou diluant pharmaceutiquement acceptable.

22. Utilisation selon la revendication 21, dans laquelle le peptide ou la protéine oncogène est un peptide ou une protéine p21-*ras* mutante, ou dans laquelle le peptide ou la protéine supprimant la tumeur est un peptide ou une protéine p53 ou un rétinoblastome.

23. Utilisation selon l'une quelconque des revendications 16 à 22, dans laquelle le cancer est choisi parmi le cancer du sein, le cancer de la prostate, le cancer du pancréas, le cancer colorectal, le cancer du poumon, un mélanome maligne, une leucémie, un lymphome, le cancer de l'ovaire, le cancer de l'utérus et les carcinomes du tractus biliaire.

**24.** Un procédé de génération de lymphocytes T capables de reconnaître et de détruire des cellules tumorales chez un mammifère, dans lequel le procédé comprend la culture d'un échantillon de lymphocytes T prélevés chez un mammifère en présence d'un peptide en une quantité suffisante pour générer des lymphocytes T spécifiques contre la télomérase, dans lequel le peptide est constitué de l'une quelconque des séquences SEQ ID n° 12 à 19, dans lequel les lymphocytes T spécifiques contre la télomérase génèrent une réponse contre un peptide constitué par l'une quelconque des séquences SEQ ID n° 12 à 19, respectivement, ou un fragment de cette séquence, long d'au moins 8 acides aminés, que l'on peut produire après traitement avec une cellule présentant un antigène.

**25.** Un lymphocyte T spécifique contre la télomérase généré par un procédé selon la revendication 24.

**Fig. 1**

**Fig. 2**

**Fig. 3**

Fig. 4

## REFERENCES CITED IN THE DESCRIPTION

### Patent documents cited in the description

- NO 9200032 W
- WO 9214756 A
- WO 9821343 A
- WO 9814593 A
- EP 03075681 A
- EP 19983141 A

### Non-patent literature cited in the description

- **BOON et al.** *Cell,* 1989, vol. 58, 293-303
- **HANS-GEORG RAMMENSEE ; THOMAS FRIEDE ; STEFAN STEVANOVIC.** *Immunogenetics,* 1995, vol. 41, 178-228
- **BARINAGA.** *Science,* 1992, vol. 257, 880-881
- **MALE et al.** Advanced Immunology. J.B. Lippincott Company, 1987
- **M.K. GJERTSEN et al.** *Int. J cancer,* 1997, vol. 72, 784
- **GREIDER ; BLACKBURN.** *Cell,* 1985, vol. 43, 405-413
- **MEYERSON et al.** *Cell,* 1990, vol. 1197, 785-795
- **NAKAMURA et al.** *Science,* 1997, vol. 277, 955-959
- **COLLINS et al.** *Cell,* 1995, vol. 81, 677-686
- **HARRINGTON et al.** *Science,* 1997, vol. 275, 973-977
- **NAKAYAMA et al.** *Cell,* 1997, vol. 88, 875-884
- **HARLEY et al.** *Cold Spring Harbor Symp. Quant. Biol.,* 1994, vol. 59, 307-315
- **KIM et al.** *Science,* 1994, vol. 266, 2011-2015
- **BROCCOLI et al.** *PNAS USA,* 1995, vol. 92, 9082-9086
- **COUNTER et al.** *Blood,* 1995, vol. 85, 2315-2320
- **HIYAMA et al.** *J. Immunol.,* 1995, vol. 155, 3711-3715
- **COUNTER et al.** *PNAS USA,* 1994, vol. 91, 2900-2904
- **SHAY ; BACHETTI.** *Eur. J. Cancer,* 1997, vol. 33, 787-791
- **KLINGELHUTZ et al.** *Nature,* 1996, vol. 380, 79-82
- **SHARMA et al.** *Ann Oncol,* 1997, vol. 8 (11), 1063-1074
- **AXELROD.** *Nature Med,* 1996, vol. 2 (2), 158-159
- **HUMINIECKI.** *Acta Biochim Pol,* 1996, vol. 43 (3), 531-538
- **MEYERSON.** *Cell,* 1990, vol. 1197, 785-795
- **SORIA ; RIXE.** *Bull Cancer,* 1997, vol. 84 (10), 963-970
- **DAHSE et al.** *Clin Chem,* 1997, vol. 43 (5), 708-714
- **MEYERSON et al.** *Cell,* 1997, vol. 90, 785-795
- **DERES.** *Nature,* 1989, 342
- **TIGHE et al.** *Immunology Today,* 1998, vol. 19 (2), 89-97
- **MANIATIS et al.** Molecular Cloning: A Laboratory Manual. Cold Spring Harbor, New York, Cold Spring Harbor Laboratory Press, 1991
- **HARLOW ; LANE.** Antibodies: A Laboratory Manual. Cold Spring Harbor, New York, Cold Spring Harbor Laboratory Press, 1988

SEQUENCE LISTING

<110> GemVax AS

<120> Antigenic Peptides Derived from Telomerase

<130> SJW/WA/46410

<140> EP 03075681.1
<141> 2003-03-10

<150> NO 19983141

<151> 1998-07-08

<160> 254

<170> PatentIn version 3.0

<210> 1
<211> 22
<212> PRT
<213> Homo sapiens
<400> 1

```
Phe Leu His Trp Leu Met Ser Val Tyr Val Val Glu Leu Leu Arg Ser
1               5               10              15

Phe Phe Tyr Val Thr Glu
            20
```

<210> 2
<211> 16
<212> PRT
<213> Homo sapiens

<400> 2

```
Glu Ala Arg Pro Ala Leu Leu Thr Ser Arg Leu Arg Phe Ile Pro Lys
1               5               10              15
```

<210> 3
<211> 16
<212> PRT
<213> Homo sapiens

<400> 3

```
        Asp Gly Leu Arg Pro Ile Val Asn Met Asp Tyr Val Val Gly Ala Arg
        1                   5                   10                  15
```

<210> 4
<211> 18
<212> PRT
<213> Homo sapiens

<400> 4

```
        Gly Val Pro Glu Tyr Gly Cys Val Val Asn Leu Arg Lys Thr Val Val
        1                   5                   10                  15

        Asn Phe
```

<210> 5
<211> 23
<212> PRT
<213> Homo sapiens
<400> 5

```
        Lys Phe Leu His Trp Leu Met Ser Val Tyr Val Val Glu Leu Leu Arg
        1                   5                   10                  15

        Ser Phe Phe Tyr Val Thr Glu
                    20
```

<210> 6
<211> 17
<212> PRT
<213> Homo sapiens
<400> 6

```
        Lys Phe Leu His Trp Leu Met Ser Val Tyr Val Val Glu Leu Leu Arg
        1                   5                   10                  15

        Ser
```

<210> 7
<211> 18
<212> PRT
<213> Homo sapiens
<400> 7

```
        Leu Met Ser Val Tyr Val Val Glu Leu Leu Arg Ser Phe Phe Tyr Val
        1                   5                   10                  15

        Thr Glu
```

<210> 8
<211> 9
<212> PRT
<213> Homo sapiens

<400> 8

```
Arg Glu Glu Ile Leu Ala Lys Phe Leu
1               5
```

<210> 9
<211> 9
<212> PRT
<213> Homo sapiens
<400> 9

```
Ile Leu Ala Lys Phe Leu His Trp Leu
1               5
```

<210> 10
<211> 9
<212> PRT
<213> Homo sapiens
<400> 10

```
Glu Leu Leu Arg Ser Phe Phe Tyr Val
1               5
```

<210> 11
<211> 9
<212> PRT
<213> Homo sapiens
<400> 11

```
Leu Met Ser Val Tyr Val Val Glu Leu
1               5
```

<210> 12
<211> 9
<212> PRT
<213> Homo sapiens
<400> 12

```
Thr Ser Arg Leu Arg Phe Ile Pro Lys
1               5
```

<210> 13
<211> 9

<212> PRT
<213> Homo sapiens
<400> 13

Leu Thr Ser Arg Leu Arg Phe Ile Pro
1                   5

<210> 14
<211> 9
<212> PRT
<213> Homo sapiens
<400> 14

Leu Leu Thr Ser Arg Leu Arg Phe Ile
1                   5

<210> 15
<211> 9
<212> PRT
<213> Homo sapiens
<400> 15

Ala Leu Leu Thr Ser Arg Leu Arg Phe
1                   5

<210> 16
<211> 9
<212> PRT
<213> Homo sapiens
<400> 16

Pro Ala Leu Leu Thr Ser Arg Leu Arg
1                   5

<210> 17
<211> 9
<212> PRT
<213> Homo sapiens
<400> 17

Arg Pro Ala Leu Leu Thr Ser Arg Leu
1                   5

<210> 18
<211> 9
<212> PRT
<213> Homo sapiens
<400> 18

```
Ala Arg Pro Ala Leu Leu Thr Ser Arg
1               5
```

<210> 19
<211> 9
<212> PRT
<213> Homo sapiens
<400> 19

```
Glu Ala Arg Pro Ala Leu Leu Thr Ser
1               5
```

<210> 20
<211> 10
<212> PRT
<213> Homo sapiens
<400> 20

```
Leu Leu Arg Ser Phe Phe Tyr Val Thr Glu
1               5                   10
```

<210> 21
<211> 8
<212> PRT
<213> Homo sapiens
<400> 21

```
Ser Arg Leu Arg Phe Ile Pro Lys
1               5
```

<210> 22
<211> 12
<212> PRT
<213> Homo sapiens
<400> 22

```
Leu Arg Pro Ile Val Asn Met Asp Tyr Val Val Gly
1               5                   10
```

<210> 23
<211> 17
<212> PRT
<213> Homo sapiens
<400> 23

```
        Pro Glu Leu Tyr Phe Val Lys Val Asp Val Thr Gly Ala Tyr Asp Thr
        1               5                   10                  15

        Ile
```

```
<210> 24
<211> 24
<212> PRT
<213> Homo sapiens
<400> 24
```

```
        Lys Ser Tyr Val Gln Cys Gln Gly Ile Pro Gln Gly Ser Ile Leu Ser
        1               5                   10                  15

        Thr Leu Leu Cys Ser Leu Cys Tyr
                    20
```

```
<210> 25
<211> 13
<212> PRT
<213> Homo sapiens
<400> 25
```

```
        Leu Leu Leu Arg Leu Val Asp Asp Phe Leu Leu Val Thr
        1               5                   10
```

```
<210> 26
<211> 11
<212> PRT
<213> Homo sapiens
<400> 26
```

```
        Gly Cys Val Val Asn Leu Arg Lys Thr Val Val
        1               5                   10
```

```
<210> 27
<211> 48
<212> PRT
<213> Homo sapiens
<400> 27
```

```
        Trp Leu Met Ser Val Tyr Val Val Glu Leu Leu Arg Ser Phe Phe Tyr
        1               5                   10                  15

        Val Thr Glu Thr Thr Phe Gln Lys Asn Arg Leu Phe Phe Tyr Arg Lys
                    20                  25                  30

        Ser Val Trp Ser Lys Leu Gln Ser Ile Gly Ile Arg Gln His Leu Lys
                    35                  40                  45
```

20

<210> 28
<211> 25
<212> PRT
<213> Homo sapiens
<400> 28


Glu Val Arg Gln His Arg Glu Ala Arg Pro Ala Leu Leu Thr Ser Arg
1               5               10              15

Leu Arg Phe Ile Pro Lys Pro Asp Gly
            20              25


<210> 29

<211> 29

<212> PRT
<213> Homo sapiens
<400> 29


Leu Arg Pro Ile Val Asn Met Asp Tyr Val Val Gly Ala Arg Thr Phe
1               5               10              15

Arg Arg Glu Lys Arg Ala Glu Arg Leu Thr Ser Arg Val
            20              25


<210> 30
<211> 34
<212> PRT
<213> Homo sapiens
<400> 30


Pro Pro Pro Glu Leu Tyr Phe Val Lys Val Asp Val Thr Gly Ala Tyr
1               5               10              15

Asp Thr Ile Pro Gln Asp Arg Leu Thr Glu Val Ile Ala Ser Ile Ile
            20              25              30

Lys Pro


<210> 31

<211> 35

<212> PRT
<213> Homo sapiens

<400> 31

EP 1 362 597 B2

```
Lys Ser Tyr Val Gln Cys Gln Gly Ile Pro Gln Gly Ser Ile Leu Ser
1               5               10              15

Thr Leu Leu Cys Ser Leu Cys Tyr Gly Asp Met Glu Asn Lys Leu Phe
            20              25              30

Ala Gly Ile
        35
```

<210> 32

<211> 17

<212> PRT
<213> Homo sapiens
<400> 32

```
Leu Leu Arg Leu Val Asp Asp Phe Leu Leu Val Thr Pro His Leu Thr
1               5               10              15

His
```

<210> 33

<211> 26

<212> PRT
<213> Homo sapiens
<400> 33

```
Ala Lys Thr Phe Leu Arg Thr Leu Val Arg Gly Val Pro Glu Tyr Gly
1               5               10              15

Cys Val Val Asn Leu Arg Lys Thr Val Val
            20              25
```

<210> 34

<211> 11

<212> PRT
<213> Homo sapiens
<400> 34

```
His Gly Leu Phe Pro Trp Cys Gly Leu Leu Leu
1               5               10
```

<210> 35
<211> 9
<212> PRT

<213> Homo sapiens
<400> 35

```
Tyr Val Val Glu Leu Leu Arg Ser Phe
1               5
```

<210> 36
<211> 9
<212> PRT
<213> Homo sapiens
<400> 36

```
Val Val Glu Leu Leu Arg Ser Phe Phe
1               5
```

<210> 37
<211> 9
<212> PRT
<213> Homo sapiens
<400> 37

```
Ser Val Tyr Val Val Glu Leu Leu Arg
1               5
```

<210> 38

<211> 9
<212> PRT
<213> Homo sapiens
<400> 38

```
Val Glu Leu Leu Arg Ser Phe Phe Tyr
1               5
```

<210> 39
<211> 9
<212> PRT
<213> Homo sapiens
<400> 39

```
Tyr Val Thr Glu Thr Thr Phe Gln Lys
1               5
```

<210> 40
<211> 9
<212> PRT
<213> Homo sapiens
<400> 40

Arg Leu Phe Phe Tyr Arg Lys Ser Val
1               5

<210> 41
<211> 9
<212> PRT
<213> Homo sapiens
<400> 41

Ser Ile Gly Ile Arg Gln His Leu Lys
1               5

<210> 42 <211> 9
<212> PRT
<213> Homo sapiens

<400> 42

Gly Val Pro Glu Tyr Gly Cys Val Val
1               5

<210> 43
<211> 9
<212> PRT
<213> Homo sapiens
<400> 43

Val Val Asn Leu Arg Lys Thr Val Val
1               5

<210> 44
<211> 9
<212> PRT
<213> Homo sapiens
<400> 44

Gly Leu Phe Pro Trp Cys Gly Leu Leu
1               5

<210> 45
<211> 9
<212> PRT
<213> Homo sapiens
<400> 45

```
Arg Pro Ile Val Asn Met Asp Tyr Val
1               5
```

<210> 46
<211> 9
<212> PRT
<213> Homo sapiens
<400> 46

```
Leu Arg Pro Ile Val Asn Met Asp Tyr
1               5
```

<210> 47
<211> 9
<212> PRT
<213> Homo sapiens
<400> 47

```
Tyr Val Val Gly Ala Arg Thr Phe Arg
1               5
```

<210> 48
<211> 9
<212> PRT
<213> Homo sapiens
<400> 48

```
Ala Arg Thr Phe Arg Arg Glu Lys Arg
1               5
```

<210> 49

<211> 9

<212> PRT

<213> Homo sapiens

<400> 49

```
Val Val Gly Ala Arg Thr Phe Arg Arg
1               5
```

<210> 50
<211> 9
<212> PRT
<213> Homo sapiens
<400> 50

Gly Ala Arg Thr Phe Arg Arg Glu Lys
1               5

<210> 51
<211> 9
<212> PRT
<213> Homo sapiens
<400> 51

Ala Arg Thr Phe Arg Arg Glu Lys Pro
1               5

<210> 52
<211> 9
<212> PRT
<213> Homo sapiens
<400> 52

Pro Pro Glu Leu Tyr Phe Val Lys Val
1               5

<210> 53
<211> 9
<212> PRT
<213> Homo sapiens
<400> 53

Glu Leu Tyr Phe Val Lys Val Asp Val
1               5

<210> 54
<211> 9
<212> PRT
<213> Homo sapiens
<400> 54

Phe Val Lys Val Asp Val Thr Gly Ala
1               5

<210> 55
<211> 9
<212> PRT
<213> Homo sapiens
<400> 55

```
Ile Pro Gln Asp Arg Leu Thr Glu Val
1               5
```

<210> 56
<211> 9
<212> PRT
<213> Homo sapiens
<400> 56

```
Asp Arg Leu Thr Glu Val Ile Ala Ser
1               5
```

<210> 57

<211> 9
<212> PRT
<213> Homo sapiens
<400> 57

```
Arg Leu Thr Glu Val Ile Ala Ser Ile
1               5
```

<210> 58
<211> 10
<212> PRT

<213> Homo sapiens
<400> 58

```
Ile Pro Gln Gly Ser Ile Leu Ser Thr Leu
1               5                 10
```

<210> 59
<211> 9
<212> PRT
<213> Homo sapiens
<400> 59

```
Ile Leu Ser Thr Leu Leu Cys Ser Leu
1               5
```

<210> 60

<211> 9
<212> PRT
<213> Homo sapiens
<400> 60

```
Leu Leu Arg Leu Val Asp Asp Phe Leu
1                   5
```

<210> 61
<211> 9
<212> PRT
<213> Homo sapiens
<400> 61

```
Arg Leu Val Asp Asp Phe Leu Leu Val
1                   5
```

<210> 62
<211> 9
<212> PRT
<213> Homo sapiens
<400> 62

```
Val Pro Glu Tyr Gly Cys Val Val Asn
1                   5
```

<210> 63
<211> 10
<212> PRT
<213> Homo sapiens
<400> 63

```
Val Pro Glu Tyr Gly Cys Val Val Asn Leu
1                   5                   10
```

<210> 64
<211> 9
<212> PRT
<213> Homo sapiens
<400> 64

```
Thr Leu Val Arg Gly Val Pro Glu Tyr
1                   5
```

<210> 65
<211> 9
<212> PRT
<213> Homo sapiens
<400> 65

Phe Leu Arg Thr Leu Val Arg Gly Val
1               5

<210> 66
<211> 9
<212> PRT
<213> Homo sapiens
<400> 66


Ala Glu Val Arg Gln His Arg Glu Ala
1               5

<210> 67
<211> 9
<212> PRT
<213> Homo sapiens
<400> 67


Arg Glu Ala Gly Val Pro Leu Gly Leu
1               5

<210> 68
<211> 9
<212> PRT
<213> Homo sapiens
<400> 68


Glu Glu Ala Thr Ser Leu Glu Gly Ala
1               5

<210> 69
<211> 9
<212> PRT
<213> Homo sapiens
<400> 69


Tyr Ala Glu Thr Lys His Phe Leu Tyr
1               5

<210> 70<211> 9
<212> PRT
<213> Homo sapiens
<400> 70

```
Ile Ser Asp Thr Ala Ser Leu Cys Tyr
1               5
```

<210> 71
<211> 9
<212> PRT
<213> Homo sapiens
<400> 71

```
Asp Thr Asp Pro Arg Arg Leu Val Gln


    1               5
```

<210> 72
<211> 9
<212> PRT
<213> Homo sapiens
<400> 72

```
Ala Gln Asp Pro Pro Pro Glu Leu Tyr
1               5
```

<210> 73
<211> 9
<212> PRT
<213> Homo sapiens
<400> 73

```
Leu Thr Asp Leu Gln Pro Tyr Met Arg
1               5
```

<210> 74
<211> 9
<212> PRT
<213> Homo sapiens
<400> 74

```
Gln Ser Asp Tyr Ser Ser Tyr Ala Arg
1               5
```

<210> 75
<211> 9
<212> PRT

<213> Homo sapiens
<400> 75

Leu Leu Ala Arg Cys Ala Leu Phe Val
1     5

<210> 76
<211> 9
<212> PRT
<213> Homo sapiens
<400> 76

Trp Leu Cys His Gln Ala Phe Leu Leu
1     5

<210> 77
<211> 9
<212> PRT
<213> Homo sapiens
<400> 77

Arg Leu Val Asp Asp Phe Leu Leu Val
1     5

<210> 78

<211> 9
<212> PRT
<213> Homo sapiens
<400> 78

Leu Glu Ala Ala Ala Asn Pro Ala Leu
1     5

<210> 79
<211> 9
<212> PRT
<213> Homo sapiens
<400> 79

Leu Gln Leu Pro Phe His Gln Gln Val
1     5

<210> 80
<211> 9
<212> PRT
<213> Homo sapiens
<400> 80

```
Arg Leu Gly Pro Gln Gly Trp Arg Leu
1               5
```

<210> 81
<211> 9
<212> PRT
<213> Homo sapiens
<400> 81

```
Ser Leu Gln Glu Leu Thr Trp Lys Met
1               5
```

<210> 82
<211> 9
<212> PRT
<213> Homo sapiens
<400> 82

```
Asn Val Leu Ala Phe Gly Phe Ala Leu
1               5
```

<210> 83
<211> 9
<212> PRT
<213> Homo sapiens
<400> 83

```
Val Leu Leu Lys Thr His Cys Pro Leu
1               5
```

<210> 84
<211> 9
<212> PRT
<213> Homo sapiens
<400> 84

```
Phe Leu Leu Val Thr Pro His Leu Thr
1               5
```

<210> 85
<211> 9
<212> PRT
<213> Homo sapiens
<400> 85

```
Thr Leu Thr Asp Leu Gln Pro Tyr Met
1               5
```

<210> 86

<211> 9
<212> PRT
<213> Homo sapiens
<400> 86

```
Gln Glu Thr Ser Pro Leu Arg Asp Ala
1               5
```

<210> 87
<211> 9
<212> PRT
<213> Homo sapiens
<400> 87

```
Phe Leu Asp Leu Gln Val Asn Ser Leu
1               5
```

<210> 88
<211> 9
<212> PRT
<213> Homo sapiens
<400> 88

```
Ser Leu Asn Glu Ala Ser Ser Gly Leu
1               5
```

<210> 89
<211> 9
<212> PRT
<213> Homo sapiens
<400> 89

```
Arg Glu Val Leu Pro Leu Ala Thr Phe
1               5
```

<210> 90
<211> 9
<212> PRT
<213> Homo sapiens
<400> 90

```
Leu Leu Gly Ala Ser Val Leu Gly Leu
1               5
```

<210> 91
<211> 9
<212> PRT
<213> Homo sapiens
<400> 91

```
Val Leu Ala Phe Gly Phe Ala Leu Leu
1               5
```

<210> 92
<211> 9
<212> PRT
<213> Homo sapiens
<400> 92

```
Leu Gln Pro Tyr Met Arg Gln Phe Val
1               5
```

<210> 93
<211> 9
<212> PRT
<213> Homo sapiens
<400> 93

```
Leu Met Ser Val Tyr Val Val Glu Leu
1               5
```

<210> 94
<211> 9
<212> PRT
<213> Homo sapiens

<400> 94

```
Arg Leu Pro Gln Arg Tyr Trp Gln Met
1               5
```

<210> 95
<211> 9
<212> PRT
<213> Homo sapiens
<400> 95

```
                    Arg Gln His Ser Ser Pro Trp Gln Val
                    1               5
```

<210> 96
<211> 9
<212> PRT
<213> Homo sapiens
<400> 96

```
                    Arg Gln His Ser Ser Pro Trp Gln Val
                    1               5
```

<210> 97
<211> 9
<212> PRT
<213> Homo sapiens
<400> 97

```
                    Tyr Leu Pro Asn Thr Val Thr Asp Ala
                    1               5
```

<210> 98
<211> 9
<212> PRT
<213> Homo sapiens
<400> 98

```
                    Asn Met Arg Arg Lys Leu Phe Gly Val
                    1               5
```

<210> 99
<211> 9
<212> PRT
<213> Homo sapiens
<400> 99

```
                    Arg Leu Thr Ser Arg Val Lys Ala Leu
                    1               5
```

<210> 100
<211> 9
<212> PRT
<213> Homo sapiens
<400> 100

Leu Leu Gln Ala Tyr Arg Phe His Ala
1                   5

<210> 101
<211> 9
<212> PRT
<213> Homo sapiens
<400> 101


Leu Leu Asp Thr Arg Thr Leu Glu Val
1                   5

<210> 102
<211> 9
<212> PRT
<213> Homo sapiens

<400> 102


Tyr Met Arg Gln Phe Val Ala His Leu
1                   5

<210> 103

<211> 9
<212> PRT
<213> Homo sapiens
<400> 103


Lys Glu Gln Leu Arg Pro Ser Phe Leu
1                   5

<210> 104
<211> 9
<212> PRT
<213> Homo sapiens
<400> 104


Cys Leu Val Cys Val Pro Trp Asp Ala
1                   5

<210> 105
<211> 9
<212> PRT<213> Homo sapiens
<400> 105

```
Leu Leu Ser Ser Leu Arg Pro Ser Leu
1               5
```

<210> 106
<211> 9
<212> PRT
<213> Homo sapiens
<400> 106

```
Phe Met Cys His His Ala Val Arg Ile
1               5
```

<210> 107
<211> 9
<212> PRT
<213> Homo sapiens
<400> 107

```
Leu Gln Val Asn Ser Leu Gln Thr Val
1               5
```

<210> 108
<211> 9
<212> PRT
<213> Homo sapiens
<400> 108

```
Leu Val Ala Gln Cys Leu Val Cys Val
1               5
```

<210> 109
<211> 9
<212> PRT
<213> Homo sapiens
<400> 109

```
Cys Leu Lys Glu Leu Val Ala Arg Val
1               5
```

<210> 110
<211> 9
<212> PRT
<213> Homo sapiens
<400> 110

```
Phe Leu Arg Asn Thr Lys Lys Phe Ile
1                   5
```

<210> 111
<211> 9
<212> PRT
<213> Homo sapiens
<400> 111

```
Ala Leu Pro Ser Asp Phe Lys Thr Ile
1                   5
```

<210> 112
<211> 9
<212> PRT
<213> Homo sapiens
<400> 112

```
Val Leu Val His Leu Leu Ala Arg Cys
1                   5
```

<210> 113
<211> 9
<212> PRT
<213> Homo sapiens
<400> 113

```
Val Gln Ser Asp Tyr Ser Ser Tyr Ala
1                   5
```

<210> 114
<211> 9
<212> PRT
<213> Homo sapiens
<400> 114

```
Ser Val Trp Ser Lys Leu Gln Ser Ile
1                   5
```

<210> 115
<211> 9
<212> PRT
<213> Homo sapiens
<400> 115

```
                        Lys Leu Pro Gly Thr Thr Leu Thr Ala
                        1               5
```

<210> 116
<211> 9
<212> PRT
<213> Homo sapiens
<400> 116

```
                       Gln Leu Ser Arg Lys Leu Pro Gly Thr
                       1               5
```

<210> 117
<211> 9
<212> PRT
<213> Homo sapiens
<400> 117

```
                      Arg Glu Lys Pro Gln Gly Ser Val Ala
                      1               5
```

<210> 118
<211> 9
<212> PRT
<213> Homo sapiens
<400> 118

```
                      Gly Leu Leu Leu Asp Thr Arg Thr Leu
                      1               5
```

<210> 119
<211> 9
<212> PRT
<213> Homo sapiens
<400> 119

```
                      Trp Met Pro Gly Thr Pro Arg Arg Leu
                      1               5
```

<210> 120
<211> 9
<212> PRT
<213> Homo sapiens
<400> 120

Ser Leu Thr Gly Ala Arg Arg Leu Val
1               5

<210> 121
<211> 9
<212> PRT
<213> Homo sapiens

<400> 121

Val Val Ile Glu Gln Ser Ser Ser Leu
1               5

<210> 122
<211> 9
<212> PRT
<213> Homo sapiens
<400> 122

Leu Pro Ser Glu Ala Val Gln Trp Leu
1               5

<210> 123
<211> 9
<212> PRT
<213> Homo sapiens

<400> 123

Gln Ala Tyr Arg Phe His Ala Cys Val
1               5

<210> 124
<211> 9
<212> PRT
<213> Homo sapiens
<400> 124

Gly Leu Phe Asp Val Phe Leu Arg Phe
1               5

<210> 125
<211> 9
<212> PRT
<213> Homo sapiens
<400> 125

Lys Leu Phe Gly Val Leu Arg Leu Lys
1               5

<210> 126
<211> 9
<212> PRT
<213> Homo sapiens
<400> 126


Arg Leu Arg Glu Glu Ile Leu Ala Lys
1               5

<210> 127
<211> 9
<212> PRT
<213> Homo sapiens
<400> 127


Leu Glu Val Gln Ser Asp Tyr Ser Ser
1               5

<210> 128
<211> 9
<212> PRT
<213> Homo sapiens
<400> 128


Gly Leu Pro Ala Pro Gly Ala Arg Arg
1               5

<210> 129
<211> 9
<212> PRT
<213> Homo sapiens
<400> 129


Gly Leu Phe Pro Trp Cys Gly Leu Leu
1               5

<210> 130
<211> 9
<212> PRT
<213> Homo sapiens

<400> 130

Lys Leu Thr Arg His Arg Val Thr Tyr
1               5

<210> 131
<211> 9
<212> PRT
<213> Homo sapiens

<400> 131

Val Leu Pro Leu Ala Thr Phe Val Arg
1               5

<210> 132
<211> 9
<212> PRT
<213> Homo sapiens

<400> 132

Glu Leu Val Ala Arg Val Leu Gln Arg
1               5

<210> 133
<211> 9
<212> PRT
<213> Homo sapiens
<400> 133

Asp Pro Arg Arg Leu Val Gln Leu Leu
1               5

<210> 134
<211> 9
<212> PRT
<213> Homo sapiens
<400> 134

Phe Val Arg Ala Cys Leu Arg Arg Leu
1               5

<210> 135
<211> 9
<212> PRT
<213> Homo sapiens
<400> 135

```
Ser Val Arg Glu Ala Gly Val Pro Leu
1               5
```

<210> 136
<211> 9
<212> PRT
<213> Homo sapiens
<400> 136

```
Ala Gly Arg Asn Met Arg Arg Lys Leu
1               5
```

<210> 137
<211> 9
<212> PRT
<213> Homo sapiens
<400> 137

```
Leu Ala Arg Cys Ala Leu Phe Val Leu
1               5
```

<210> 138
<211> 9
<212> PRT
<213> Homo sapiens
<400> 138

```
Arg Pro Ala Glu Glu Ala Thr Ser Leu
1               5
```

<210> 139
<211> 9
<212> PRT
<213> Homo sapiens
<400> 139

```
Leu Pro Ser Asp Phe Lys Thr Ile Leu
1               5
```

<210> 140
<211> 9
<212> PRT
<213> Homo sapiens

<400> 140

Leu Pro Ser Glu Ala Val Gln Trp Leu
1                   5

<210> 141
<211> 9
<212> PRT
<213> Homo sapiens

<400> 141


Leu Pro Gly Thr Thr Leu Thr Ala Leu
1                   5

<210> 142
<211> 9
<212> PRT
<213> Homo sapiens
<400> 142


Arg Pro Ser Phe Leu Leu Ser Ser Leu
1                   5

<210> 143
<211> 9
<212> PRT
<213> Homo sapiens
<400> 143


Leu Pro Asn Thr Val Thr Asp Ala Leu
1                   5

<210> 144
<211> 9
<212> PRT
<213> Homo sapiens
<400> 144


Arg Glu Ala Arg Pro Ala Leu Leu Thr
1                   5

<210> 145
<211> 9
<212> PRT
<213> Homo sapiens

<400> 145

Arg Cys Arg Ala Val Arg Ser Leu Leu
1               5

<210> 146
<211> 9
<212> PRT
<213> Homo sapiens
<400> 146

Met Pro Arg Ala Pro Arg Cys Arg Ala
1               5

<210> 147
<211> 9
<212> PRT
<213> Homo sapiens
<400> 147

Gly Ile Arg Arg Asp Gly Leu Leu Leu
1               5

<210> 148
<211> 9
<212> PRT
<213> Homo sapiens
<400> 148

Val Leu Arg Leu Lys Cys His Ser Leu
1               5

<210> 149
<211> 9
<212> PRT
<213> Homo sapiens
<400> 149

Tyr Met Arg Gln Phe Val Ala His Leu
1               5

<210> 150
<211> 9
<212> PRT
<213> Homo sapiens
<400> 150

```
Ser Leu Arg Thr Ala Gln Thr Gln Leu
1               5
```

<210> 151
<211> 9
<212> PRT
<213> Homo sapiens
<400> 151

```
Gln Met Arg Pro Leu Phe Leu Glu Leu
1               5
```

<210> 152
<211> 9
<212> PRT
<213> Homo sapiens
<400> 152

```
Glu Glu Asp Thr Asp Pro Arg Arg Leu
1               5
```

<210> 153
<211> 9
<212> PRT
<213> Homo sapiens
<400> 153

```
Phe Val Gln Met Pro Ala His Gly Leu
1               5
```

<210> 154
<211> 9
<212> PRT
<213> Homo sapiens
<400> 154

```
His Ala Ser Gly Pro Arg Arg Arg Leu
1               5
```

<210> 155
<211> 9
<212> PRT
<213> Homo sapiens
<400> 155

```
Val Val Ile Glu Gln Ser Ser Ser Leu
1               5
```

<210> 156
<211> 9
<212> PRT
<213> Homo sapiens
<400> 156

```
Arg Val Ile Ser Asp Thr Ala Ser Leu
1               5
```

<210> 157
<211> 9
<212> PRT
<213> Homo sapiens
<400> 157

```
Cys Val Pro Ala Ala Glu His Arg Leu
1               5
```

<210> 158
<211> 9
<212> PRT
<213> Homo sapiens
<400> 158

```
Arg Val Lys Ala Leu Phe Ser Val Leu
1               5
```

<210> 159
<211> 9
<212> PRT
<213> Homo sapiens
<400> 159

```
Asn Val Leu Ala Phe Gly Phe Ala Leu
1               5
```

<210> 160
<211> 9
<212> PRT
<213> Homo sapiens

<400> 160

```
            Leu Val Ala Arg Val Leu Gln Arg Leu
            1               5
```

<210> 161
<211> 9
<212> PRT
<213> Homo sapiens
<400> 161

```
            Phe Ala Gly Ile Arg Arg Asp Gly Leu
            1               5
```

<210> 162
<211> 9
<212> PRT
<213> Homo sapiens
<400> 162

```
            His Ala Gln Cys Pro Tyr Gly Val Leu
            1               5
```

<210> 163
<211> 9
<212> PRT
<213> Homo sapiens
<400> 163

```
            Arg Ala Gln Asp Pro Pro Pro Glu Leu
            1               5
```

<210> 164
<211> 9
<212> PRT
<213> Homo sapiens
<400> 164

```
            Ala Tyr Arg Phe His Ala Cys Val Leu
            1               5
```

<210> 165
<211> 9
<212> PRT
<213> Homo sapiens
<400> 165

His Ala Lys Leu Ser Leu Gln Glu Leu
1                   5

<210> 166
<211> 9
<212> PRT
<213> Homo sapiens
<400> 166

Gly Ala Lys Gly Ala Ala Gly Pro Leu
1                   5

<210> 167
<211> 9
<212> PRT
<213> Homo sapiens
<400> 167

Thr Ala Ser Leu Cys Tyr Ser Ile Leu
1                   5

<210> 168
<211> 9
<212> PRT
<213> Homo sapiens

<400> 168

Ala Pro Arg Cys Arg Ala Val Arg Ser
1                   5

<210> 169
<211> 9
<212> PRT
<213> Homo sapiens
<400> 169

Gly Ala Arg Arg Leu Val Glu Thr Ile
1                   5

<210> 170
<211> 9
<212> PRT
<213> Homo sapiens

<400> 170

Ala Gln Cys Pro Tyr Gly Val Leu Leu
1               5

<210> 171
<211> 9
<212> PRT
<213> Homo sapiens
<400> 171

His Ala Lys Thr Phe Leu Arg Thr Leu
1               5

<210> 172
<211> 9
<212> PRT
<213> Homo sapiens

<400> 172

Glu Ala Thr Ser Leu Glu Gly Ala Leu
1               5

<210> 173
<211> 9
<212> PRT
<213> Homo sapiens
<400> 173

Lys Ala Lys Asn Ala Gly Met Ser Leu
1               5

<210> 174
<211> 9
<212> PRT
<213> Homo sapiens
<400> 174

Ala Gln Thr Gln Leu Ser Arg Lys Leu
1               5

<210> 175
<211> 9
<212> PRT
<213> Homo sapiens

<400> 175

Ala Gly Ile Arg Arg Asp Gly Leu Leu
1               5

<210> 176
<211> 9
<212> PRT
<213> Homo sapiens
<400> 176

Val Leu Arg Leu Lys Cys His Ser Leu
1               5

<210> 177
<211> 9
<212> PRT
<213> Homo sapiens
<400> 177

Ile Leu Lys Ala Lys Asn Ala Gly Met
1               5

<210> 178
<211> 9
<212> PRT
<213> Homo sapiens
<400> 178

Asp Pro Arg Arg Leu Val Gln Leu Leu
1               5

<210> 179
<211> 9
<212> PRT
<213> Homo sapiens
<400> 179

Gly Ala Lys Gly Ala Ala Gly Pro Leu
1               5

<210> 180
<211> 9
<212> PRT
<213> Homo sapiens

<400> 180

EP 1 362 597 B2

Phe Ala Gly Ile Arg Arg Asp Gly Leu
1                   5

<210> 181
<211> 9
<212> PRT
<213> Homo sapiens
<400> 181

Gly Ala Arg Arg Arg Gly Gly Ser Ala
1                   5

<210> 182
<211> 9
<212> PRT
<213> Homo sapiens

<400> 182

His Ala Lys Thr Phe Leu Arg Thr Leu
1                   5

<210> 183
<211> 9
<212> PRT
<213> Homo sapiens

<400> 183

His Ala Lys Leu Ser Leu Gln Glu Leu
1                   5

<210> 184
<211> 9
<212> PRT
<213> Homo sapiens
<400> 184

Leu Ala Arg Cys Ala Leu Phe Val Leu
1                   5

<210> 185
<211> 9
<212> PRT
<213> Homo sapiens
<400> 185

52

Glu His Arg Leu Arg Glu Glu Ile Leu
1               5

<210> 186
<211> 9
<212> PRT
<213> Homo sapiens

<400> 186

Asn Met Arg Arg Lys Leu Phe Gly Val
1               5

<210> 187
<211> 9
<212> PRT
<213> Homo sapiens
<400> 187

Cys Ala Arg Glu Lys Pro Gln Gly Ser
1               5

<210> 188
<211> 9
<212> PRT
<213> Homo sapiens
<400> 188

Leu Thr Arg His Arg Val Thr Tyr Val
1               5

<210> 189
<211> 9
<212> PRT
<213> Homo sapiens
<400> 189

Arg Arg Phe Leu Arg Asn Thr Lys Lys
1               5

<210> 190
<211> 9
<212> PRT

<213> Homo sapiens
<400> 190


Arg Arg Asp Gly Leu Leu Leu Arg Leu
1               5


<210> 191
<211> 9
<212> PRT
<213> Homo sapiens
<400> 191


Arg Arg Glu Lys Arg Ala Glu Arg Leu
1               5


<210> 192
<211> 9
<212> PRT
<213> Homo sapiens
<400> 192


Arg Arg Leu Val Glu Thr Ile Phe Leu
1               5


<210> 193
<211> 9
<212> PRT
<213> Homo sapiens
<400> 193


Leu Arg Phe Met Cys His His Ala Val
1               5


<210> 194
<211> 9
<212> PRT
<213> Homo sapiens
<400> 194


Arg Arg Tyr Ala Val Val Gln Lys Ala
1               5


<210> 195

<211> 9
<212> PRT
<213> Homo sapiens

<400> 195

Lys Arg Ala Glu Arg Leu Thr Ser Arg
1               5

<210> 196
<211> 9
<212> PRT
<213> Homo sapiens

<400> 196

Arg Arg Lys Leu Phe Gly Val Leu Arg
1               5

<210> 197
<211> 9
<212> PRT
<213> Homo sapiens
<400> 197

Arg Arg Arg Gly Gly Ser Ala Ser Arg
1               5

<210> 198
<211> 9
<212> PRT
<213> Homo sapiens
<400> 198

Arg Arg Leu Pro Arg Leu Pro Gln Arg
1               5

<210> 199
<211> 9
<212> PRT
<213> Homo sapiens
<400> 199

Arg Arg Leu Gly Pro Gln Gly Trp Arg
1               5

<210> 200
<211> 9

<212> PRT
<213> Homo sapiens

<400> 200

Leu Arg Gly Ser Gly Ala Trp Gly Leu
1               5

<210> 201
<211> 9
<212> PRT
<213> Homo sapiens
<400> 201

His Arg Glu Ala Arg Pro Ala Leu Leu
1               5

<210> 202
<211> 9
<212> PRT
<213> Homo sapiens

<400> 202

Val Arg Arg Tyr Ala Val Val Gln Lys
1               5

<210> 203
<211> 9
<212> PRT
<213> Homo sapiens
<400> 203

Ala Arg Thr Ser Ile Arg Ala Ser Leu
1               5

<210> 204
<211> 9
<212> PRT
<213> Homo sapiens
<400> 204

His Arg Val Thr Tyr Val Pro Leu Leu
1               5

<210> 205
<211> 9

<212> PRT
<213> Homo sapiens
<400> 205

```
Leu Arg Ser His Tyr Arg Glu Val Leu
1               5
```

<210> 206
<211> 9
<212> PRT
<213> Homo sapiens
<400> 206

```
Met Arg Pro Leu Phe Leu Glu Leu Leu
1               5
```

<210> 207
<211> 9
<212> PRT
<213> Homo sapiens
<400> 207

```
His Arg Ala Trp Arg Thr Phe Val Leu
1               5
```

<210> 208
<211> 9
<212> PRT
<213> Homo sapiens
<400> 208

```
Met Arg Arg Lys Leu Phe Gly Val Leu
1               5
```

<210> 209
<211> 9
<212> PRT
<213> Homo sapiens
<400> 209

```
Leu Arg Leu Val Asp Asp Phe Leu Leu
1               5
```

<210> 210
<211> 9
<212> PRT
<213> Homo sapiens
<400> 210

```
                         Leu Arg Arg Val Gly Asp Asp Val Leu
                         1               5
```

<210> 211
<211> 9
<212> PRT
<213> Homo sapiens
<400> 211

```
                          Tyr Arg Lys Ser Val Trp Ser Lys Leu
                          1               5
```

<210> 212
<211> 9
<212> PRT
<213> Homo sapiens
<400> 212

```
                         Gln Arg Leu Cys Glu Arg Gly Ala Lys
                         1               5
```

<210> 213
<211> 9
<212> PRT
<213> Homo sapiens
<400> 213

```
                         Phe Arg Ala Leu Val Ala Gln Cys Leu
                         1               5
```

<210> 214
<211> 9
<212> PRT
<213> Homo sapiens
<400> 214

```
                         Ser Arg Lys Leu Pro Gly Thr Thr Leu
                         1               5
```

<210> 215
<211> 9
<212> PRT
<213> Homo sapiens
<400> 215

```
                        Leu Arg Arg Leu Val Pro Pro Gly Leu
                        1               5
```

<210> 216
<211> 9
<212> PRT
<213> Homo sapiens
<400> 216

```
                        Arg Arg Ser Pro Gly Val Gly Cys Val
                        1               5
```

<210> 217
<211> 9
<212> PRT
<213> Homo sapiens
<400> 217

```
                        Arg Arg Val Gly Asp Asp Val Leu Val
                        1               5
```

<210> 218
<211> 9
<212> PRT
<213> Homo sapiens
<400> 218

```
                        Val Arg Gly Cys Ala Trp Leu Arg Arg
                        1               5
```

<210> 219
<211> 9
<212> PRT
<213> Homo sapiens
<400> 219

```
                        Val Arg Ser Leu Leu Arg Ser His Tyr
                        1               5
```

<210> 220
<211> 9
<212> PRT
<213> Homo sapiens
<400> 220

```
                    Gly Asp Met Glu Asn Lys Leu Phe Ala
                    1               5
```

<210> 221
<211> 9
<212> PRT
<213> Homo sapiens

<400> 221

```
                    Ser Arg Ser Leu Pro Leu Pro Lys Arg
                    1               5
```

<210> 222
<211> 9
<212> PRT
<213> Homo sapiens
<400> 222

```
                    Ile Arg Ala Ser Leu Thr Phe Asn Arg
                    1               5
```

<210> 223
<211> 9
<212> PRT
<213> Homo sapiens
<400> 223

```
                    Leu Arg Glu Glu Ile Leu Ala Lys Phe
                    1               5
```

<210> 224
<211> 9
<212> PRT
<213> Homo sapiens
<400> 224

```
                    Ile Arg Arg Asp Gly Leu Leu Leu Arg
                    1               5
```

<210> 225
<211> 9
<212> PRT
<213> Homo sapiens

<400> 225

```
Gln Arg Gly Asp Pro Ala Ala Phe Arg
1               5
```

<210> 226
<211> 9
<212> PRT
<213> Homo sapiens
<400> 226

```
Cys Glu Arg Gly Ala Lys Asn Val Leu
1               5
```

<210> 227
<211> 9
<212> PRT
<213> Homo sapiens
<400> 227

```
Ala Arg Arg Leu Val Glu Thr Ile Phe
1               5
```

<210> 228
<211> 9
<212> PRT
<213> Homo sapiens
<400> 228

```
Asp Asp Val Leu Val His Leu Leu Ala
1               5
```

<210> 229
<211> 9
<212> PRT
<213> Homo sapiens
<400> 229

```
Leu Arg Pro Ser Leu Thr Gly Ala Arg
1               5
```

<210> 230
<211> 9
<212> PRT
<213> Homo sapiens
<400> 230

```
Leu Arg Leu Lys Cys His Ser Leu Phe
1               5
```

<210> 231
<211> 9
<212> PRT
<213> Homo sapiens
<400> 231

```
Phe Arg Arg Glu Lys Arg Ala Glu Arg
1               5
```

<210> 232
<211> 9
<212> PRT
<213> Homo sapiens
<400> 232

```
Ala Arg Gly Gly Pro Pro Glu Ala Phe
1               5
```

<210> 233
<211> 9
<212> PRT
<213> Homo sapiens
<400> 233

```
Cys Arg Ala Val Arg Ser Leu Leu Arg
1               5
```

<210> 234
<211> 9
<212> PRT
<213> Homo sapiens
<400> 234

```
Gly Arg Thr Arg Gly Pro Ser Asp Arg
1               5
```

<210> 235
<211> 9
<212> PRT
<213> Homo sapiens
<400> 235

```
Arg Arg Arg Leu Gly Cys Glu Arg Ala
1               5
```

<210> 236
<211> 9
<212> PRT
<213> Homo sapiens
<400> 236

```
Leu Arg Glu Leu Ser Glu Ala Glu Val
1               5
```

<210> 237
<211> 9
<212> PRT
<213> Homo sapiens

<400> 237

```
Ala Arg Cys Ala Leu Phe Val Leu Val
1               5
```

<210> 238
<211> 9
<212> PRT
<213> Homo sapiens

<400> 238

```
Arg Pro Ala Glu Glu Ala Thr Ser Leu
1               5
```

<210> 239
<211> 9
<212> PRT
<213> Homo sapiens
<400> 239

```
Asp Pro Arg Arg Leu Val Gln Leu Leu
1               5
```

<210> 240
<211> 9
<212> PRT
<213> Homo sapiens
<400> 240

```
Arg Pro Ser Phe Leu Leu Ser Ser Leu
1               5
```

<210> 241
<211> 9
<212> PRT
<213> Homo sapiens
<400> 241

```
Leu Pro Ser Glu Ala Val Gln Trp Leu
1               5
```

<210> 242
<211> 9
<212> PRT
<213> Homo sapiens
<400> 242

```
Tyr Glu Arg Ala Arg Arg Pro Gly Leu
1               5
```

<210> 243
<211> 9
<212> PRT
<213> Homo sapiens

<400> 243

```
Leu Pro Ser Asp Phe Lys Thr Ile Leu
1               5
```

<210> 244
<211> 9
<212> PRT
<213> Homo sapiens
<400> 244

```
Arg Pro Pro Pro Ala Ala Pro Ser Phe
1               5
```

<210> 245
<211> 9
<212> PRT
<213> Homo sapiens
<400> 245

```
Leu Pro Arg Leu Pro Gln Arg Tyr Trp
1               5
```

<210> 246
<211> 9
<212> PRT
<213> Homo sapiens
<400> 246

```
Leu Pro Asn Thr Val Thr Asp Ala Leu
1               5
```

<210> 247
<211> 9
<212> PRT
<213> Homo sapiens
<400> 247

```
Leu Pro Gly Thr Thr Leu Thr Ala Leu
1               5
```

<210> 248
<211> 9
<212> PRT
<213> Homo sapiens

<400> 248

```
Leu Ala Lys Phe Leu His Trp Leu Met
1               5
```

<210> 249
<211> 9
<212> PRT
<213> Homo sapiens
<400> 249

```
Lys Ala Lys Asn Ala Gly Met Ser Leu
1               5
```

<210> 250
<211> 9
<212> PRT
<213> Homo sapiens

<400> 250

```
Gly Ser Arg His Asn Glu Arg Arg Phe
1               5
```

<210> 251
<211> 9
<212> PRT
<213> Homo sapiens
<400> 251

```
Lys Ala Leu Phe Ser Val Leu Asn Tyr
1               5
```

<210> 252
<211> 9
<212> PRT
<213> Homo sapiens

<400> 252

```
Ser Pro Leu Arg Asp Ala Val Val Ile
1               5
```

<210> 253
<211> 9
<212> PRT
<213> Homo sapiens
<400> 253

```
Arg Ala Gln Asp Pro Pro Pro Glu Leu
1               5
```

<210> 254
<211> 9
<212> PRT
<213> Homo sapiens

<400> 254

```
Met Pro Ala His Gly Leu Phe Pro Trp
1               5
```